(19) Europäisches Patentamt
European Patent Office
Office européen des brevets

(11) EP 2 061 461 B1

(12) # EUROPEAN PATENT SPECIFICATION

(45) Date of publication and mention of the grant of the patent:
**23.12.2009 Bulletin 2009/52**

(21) Application number: **07803325.5**

(22) Date of filing: **07.09.2007**

(51) Int Cl.:
***A61K 31/435*** $^{(2006.01)}$ ***A61P 25/24*** $^{(2006.01)}$
***C07D 221/04*** $^{(2006.01)}$

(86) International application number:
**PCT/EP2007/059380**

(87) International publication number:
**WO 2008/031771 (20.03.2008 Gazette 2008/12)**

(54) **3-AZABICYCLO[4.1.0]HEPTANE DERIVATIVES FOR THE TREATMENT OF DEPRESSION**

3-AZABICYCLO[4.1.0]HEPTAN-DERIVATE ZUR BEHANDLUNG VON DEPRESSIONEN

DERIVES DU 3-AZABICYCLYO[4.1.0]HEPTANE POUR LE TRAITEMENT DE LA DEPRESSION

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**HR**

(30) Priority: **11.09.2006 GB 0617867**

(43) Date of publication of application:
**27.05.2009 Bulletin 2009/22**

(73) Proprietor: **Glaxo Group Limited**
**Greenford, Middlesex UB6 0NN (GB)**

(72) Inventors:
- **DI FABIO, Romano**
  **I-37135 Verona (IT)**
- **MICHELI, Fabrizio**
  **I-37135 Verona (IT)**
- **TEDESCO, Giovanna**
  **I-37135 Verona (IT)**
- **TERRENI, Silvia**
  **I-37135 Verona (IT)**

(74) Representative: **Sewell, Richard Charles et al**
**GlaxoSmithKline**
**Corporate Intellectual Property**
**CN925.1**
**980 Great West Road**
**Brentford, Middlesex TW8 9GS (GB)**

(56) References cited:
**WO-A-92/06079 US-A1- 2002 016 337**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

The present invention relates to compounds defined by formula (I), pharmaceutical compositions containing them and for their use in therapy, as serotonin (5-HT), dopamine (DA) and norepinephrine (NE), re-uptake inhibitors, for the treatment of depression.

Brain tissue is constituted of neuronal cells which are able to communicate with each other via specific cellular structures named synapses. The exchange of signals between neurons in the synapses happens through neurochemical messengers named neurotransmitters, acting on specific target protein molecules, both post and pre-synaptic, referred to as receptors. Monoamines represent a family of small neurotransmitter molecules sharing common chemical features, and include serotonin (5-HT), dopamine (DA) and norepinephrine (NE).

Monoamine neurotransmitters are released into the synaptic cleft between neurons and interact with receptors present on the membrane of the target cells. The switch of the neurochemical signal occurs mainly by removal of the neurotransmitter molecules through other protein molecules referred to as monoamine transporters (SERT for 5-HT, DAT for DA and NET for NE). Transporters are able to bind neurotransmitter molecules and move them into the presynaptic terminals, this cellular mechanism referred to as re-uptake. Pharmacological inhibition of the re-uptake process can cause an increase of monoamine at synaptic level and as a consequence an enhancement of the physiological activity of neurotransmitters.

Serotonergic neurotransmission in the brain is mediated by a large family of receptors comprising both the G-protein coupled receptors and ligand-gated ion channels including 14 subtypes, and is involved in a vast variety of physiologic functions.

Compounds endowed of inhibitory properties at the SERT are predicted to have the ability to treat in mammals, including humans, a variety of disorders associated with this neural system, for example eating disorders, major depression and mood disorders, obsessive compulsive disorders, panic disorders, alcoholism, pain, memory deficits and anxiety. Included among these disorders are disorders related to depression, such as pseudodementia or Ganser's syndrome, migraine pain, bulimia, obesity, pre-menstrual syndrome or late luteal phase syndrome, tobacco abuse, panic disorder, post-traumatic syndrome, memory loss, dementia of ageing, acquired immunodeficiency syndrome dementia complex, memory dysfunction in ageing, social phobia, attention deficit hyperactivity disorder, chronic fatigue syndrome, premature ejaculation, erectile difficulty, anorexia nervosa, disorders of sleep, autism, mutism or trichotillomania.

Major depression is an affective disorder, or disorder of mood, characterized by several symptoms including feeling of profound sadness, worthlessness, despair and loss of interest in all pleasures (anhedonia), recurrent thoughts of death, mental slowing, loss of energy, an inability to take decision, often associated with anxiety and agitation. These symptoms are persistent and can range from mild to severe.

The pathophysiology of major depression is poorly understood being a multifactorial syndrome and, due to this, several neurotransmitter systems have been implicated. However, it is generally believed that the disorder stems from a decrease in the synaptic concentration of monoamine neurotransmitters, mainly NE and 5-HT, in critical brain areas, leading to the "monoamine theory" of depression.

Several lines of preclinical and clinical evidence indicate that an enhancement of serotonin-mediated neurotransmission might be effective in the treatment of major depression and actually the selective serotonin re-uptake inhibitors (SSRIs) have come to dominate the therapy of depression over the last two decades. Fluoxetine, the first SSRI to be introduced, is the prototype of this group. Other members include Paroxetine, Sertraline, Fluvoxamine, Citalopram. However, it is not clear exactly how these agents act to relieve depression. As with other classes of antidepressant, there is a lag of several weeks before the onset of the mood-elevating effect, despite the rapid blockade of the serotonin re-uptake. It is presumed that secondary adaptive changes must occur at serotonergic synapses after chronic administration of SSRIs i.e. down-regulation of release-regulating autoreceptors and increased neurotransmitter release. The delayed onset of anti-depressant effect is considered to be a serious drawback to currently used SSRIs. Moreover, although there is generally good tolerability of SSRIs, the elevation of 5-HT levels at central and peripheral synapses leads to stimulation of receptor subtypes like $5\text{-}HT_{2C}$ and $5\text{-}HT_3$, which contributes to agitation and restless, along with gastrointestinal and sexual side-effects.

The success of the SSRIs rekindled interest in the development of selective norepinephrine re-uptake inhibitors (SNRIs) as potential antidepressants. A number of such compounds have been synthesized, e.g. Nisoxetine, Maprotiline, Tomoxetine and Reboxetine. Furthermore, many compounds, including old tricyclic antidepressants, have a mixed NET and SERT inhibition profile, like Imipramine and Amitriptyline (with SERT potency > NET) and Desipramine, Nortriptyline, and Protriptyline (NET potency > SERT).

The pharmacological manipulation of the DAT can in principle have the ability to elevate DA levels in the mesolimbic system, reversing the anhedonia that is a core symptom of major depression. A DAT inhibition component, in combination with a blockade of SERT and NET, can also have the ability to improve the lack of motivation and attention and enhance cognitive deficits seen in depressed patients. On the other hand, blockade of DAT has to be carefully managed in order to avoid potential reinforcing effects and abuse liability. However compounds with DAT inhibition in

their pharmacology, such as Dexmethylphenidate, Methylphenidate and Bupropion, have been successfully marketed. Clinical studies indicate that patients with poor response to SSRIs benefit from combination therapy with agents that enhance dopaminergic tone. As a result, compounds with a strong SERT inhibiting activity combined with a well balanced NET blockade and moderate DAT inhibiting activity may therefore provide a replacement for current combination therapies for treating unresponsive patients, providing greater efficacy and therapeutic flexibility with a more rapid onset of anti-depressant effect.

Due to their valuable DAT inhibition, the compounds of the present invention are considered useful for the treatment of Parkinsonism, depression, obesity, narcolepsy, drug addiction or misuse, including cocaine abuse, attention-deficit hyperactivity disorders, Gilles de la Tourettes disease and senile dementia. Dopamine re-uptake inhibitors enhance indirectly via the dopamine neurones the release of acetylcholine and are therefore also useful for the treatment of memory deficits, e.g. in Alzheimers disease, presenile dementia, memory dysfunction in ageing, and chronic fatigue syndrome. Noradrenaline re-uptake inhibitors are considered useful for enhancing attention, alertness, arousal, vigilance and for treating depression.

The object of the present invention is to provide novel compounds which are serotonin (5-HT), dopamine (DA) and norepinephrine (NE), re-uptake inhibitors.

In a first aspect, the present invention provides a compound of formula (I) or a salt or solvate thereof:

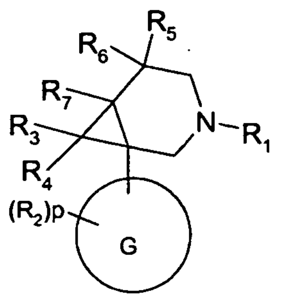

(I)

wherein

- G is selected from a group consisting of: phenyl, a 5- or 6-membered monocyclic heteroaryl group, or a 8- to 11-membered heteroaryl bicyclic group; such G may be substituted by $(R_2)_p$, which can be the same or different;
- $R_1$ is hydrogen or $C_{1-4}$ alkyl;
- $R_2$ is halogen, hydroxy, cyano, $C_{1-4}$alkyl, halo$C_{1-4}$alkyl, $C_{1-4}$alkoxy, halo$C_{1-4}$alkoxy, $C_{1-4}$alkanoyl and $SF_5$; or corresponds to $R_8$;
- $R_5$ is hydrogen or $C_{1-4}$alkyl;
- $R_6$ is hydrogen or $C_{1-4}$ alkyl;
- $R_7$ is selected in the group consisting of: hydrogen, fluorine, and $C_{1-4}$ alkyl; or corresponds to X, $X_1$, $X_2$ or $X_3$; wherein
- X corresponds to:

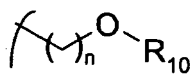

- $X_1$ corresponds to:

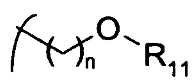

- $X_2$ corresponds to:

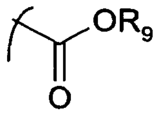

$X_3$ corresponds to:

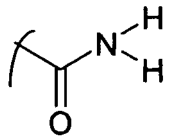

- $R_3$ is hydrogen or $C_{14}$ alkyl; or corresponds to X or $X_1$;
- $R_4$ is hydrogen or $C_{1-4}$ alkyl; or corresponds to X or $X_1$;
- $R_8$ is a 5-6 membered heterocycle group, which may be substituted by one or two substituents selected from a group consisting of: halogen, cyano, $C_{1-4}$alkyl, halo$C_{1-4}$alkyl, $C_{1-4}$alkoxy and $C_{1-4}$alkanoyl;
- $R_9$ is $C_{1-4}$alkyl;
- $R_{10}$ is hydrogen , $C_{1-4}$alkyl, $C_{3-6}$cycloalkyl or $C_{3-6}$cycloalkyl$C_{1-3}$alkyl;
- $R_{11}$ is halo$C_{1-2}$alkyl;
- p is an integer from 0 to 5;
- n is 1 or 2.

In one embodiment, the present invention provides a compound of formula (IF) or a pharmaceutically acceptable salt or a solvate thereof:

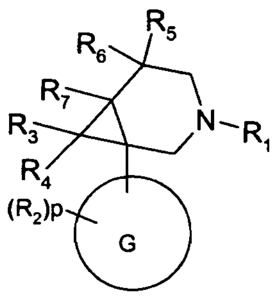

(IF)

wherein

- G is selected from a group consisting of: phenyl, a 5- or 6-membered heteroaromatic group, or a 8- to 11-membered heteroaryl bicyclic group; such G may be substituted by
- $(R_2)_p$, which can be the same or different;
- $R_1$ is hydrogen or $C_{1-4}$ alkyl;
- $R_2$ is hydrogen, halogen, hydroxy, cyano, $C_{1-4}$alkyl, halo$C_{1-4}$alkyl, $C_{1-4}$alkoxy, halo$C_{1-4}$alkoxy, $C_{1-4}$alkanoyl and $SF_5$; or corresponds to $R_8$;
- $R_3$ is hydrogen or $C_{1-4}$alkyl; or corresponds to X or $X_1$;
- $R_4$ is hydrogen or $C_{1-4}$ alkyl; or corresponds to X or $X_1$;
- $R_5$ is hydrogen or $C_{1-4}$alkyl;
- $R_6$ is hydrogen or $C_{1-4}$alkyl;
- $R_7$ is selected in the group consisting of: hydrogen, fluorine, and $C_{1-4}$ alkyl; or corresponds to X, $X_1$, $X_2$ or $X_3$; wherein
- X corresponds to:

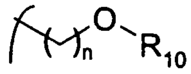

$X_1$ corresponds to:

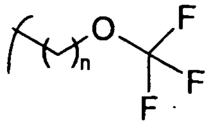

$X_2$ corresponds to:

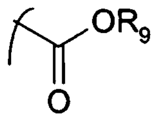

$X_3$ corresponds to:

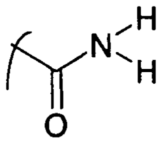

$R_8$ is a 5-6 membered heterocycle group, which may be substituted by one or two substituents selected from a group consisting of: halogen, cyano, $C_{1-4}$alkyl, halo$C_{1-4}$alkyl, $C_{1-4}$alkoxy and $C_{1-4}$alkanoyl;
$R_9$ is $C_{1-4}$alkyl;
$R_{10}$ is hydrogen , $C_{1-4}$alkyl, $C_{3-6}$cycloalkyl or $C_{3-6}$cycloalkyl$C_{1-3}$alkyl;
p is an integer from 0 to 5;
n is 1 or 2.

Because of the presence of the fused cyclopropane ring, compounds of formula (I) are believed to have a "*cis*" disposition of the substituents (both groups G and $R_7$ linked to the bicyclic ring system are on the same face of this bicyclic ring system).

It will be appreciated that compounds of formula (I) possess at least two stereogenic centers, namely at position 1 and 6 in the 3-azabicyclo[4.1.0]heptane portion of the molecule. Thus, the compounds may exist in two stereoisomers which are enantiomers with respect to the stereogenic centers in the cyclopropane ring. It will also be appreciated, in common with most biologically active molecules that the level of biological activity may vary between the individual stereoisomers of a given molecule. Also disclosed are all individual stereoisomers (diastereoisomers and enantiomers) and all mixtures thereof, including racemic mixtures, which demonstrate appropriate biological activity with reference to the procedures described herein.

In one embodiment of the present invention compounds of formula (I)' are provided which correspond to the compounds of formula (I), or pharmaceutically acceptable salts or solvates thereof, having "cis" disposition, represented by the bold highlight of the two bonds near the cyclopropyl moiety:

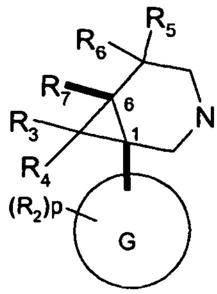

(I)'

wherein $R_1$, $R_2$, $R_3$, $R_4$, $R_5$, $R_6$, $R_7$, p and G are defined as above for compounds of formula (I).

From now on throughout the document, the symbol ' (prime) is used to identify compounds having "cis" disposition for the bonds bearing groups G and $R_7$, represented by the bold highlight of the two bonds near the cyclopropyl moiety.

In one embodiment of the present invention, the bold highlight of the two bonds near the cyclopropyl moiety bearing groups G and $R_7$, indicate, mixtures (including racemic mixtures) of those cis isomers.

In compounds of formula (I)' there are at least two stereogenic centers, which are located in the cyclopropane portion, as depicted below; through optical resolution of a mixture containing the two stereoisomers which are enantiomers with respect to the stereogenic centers at positions named 1 and 6, steroisomers of compounds of formula (I)' having a single absolute configuration at stereogenic centers named 1 and 6, may be obtained as shown in the scheme below:

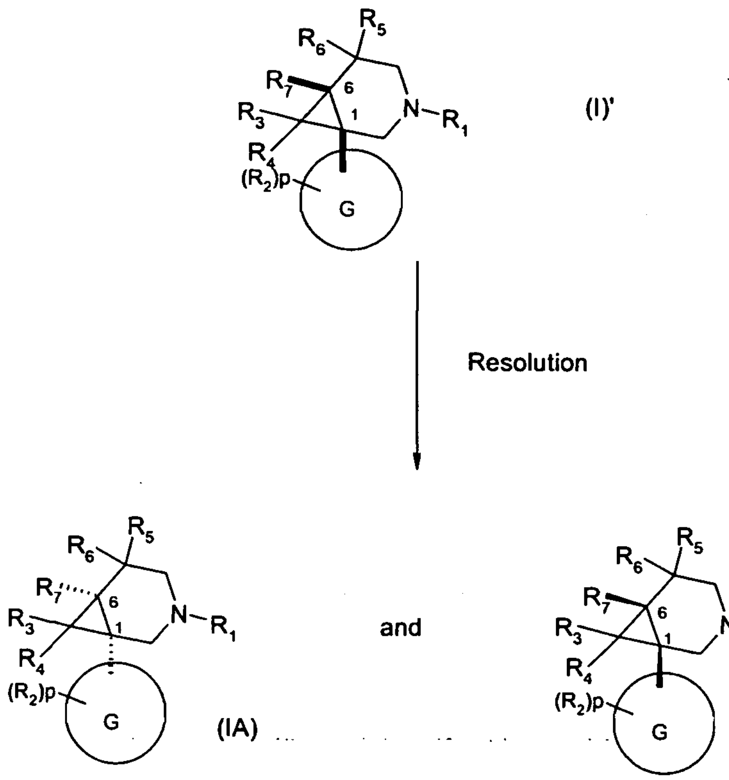


(I)'

(IA) and (IB)

Absolute configuration of stereogenic centers at position named 1 and 6 may be assigned using Cahn-Ingold-Prelog nomenclature based on groups' priorities.

In another embodiment of the present invention compounds of formula (I)" are provided which correspond to the compounds of formula (I), or pharmaceutically acceptable salts, or solvates thereof, as stereochemical isomers having a "cis" disposition for bonds bearing groups G and $R_7$, and a single but unknown configuration at stereogenic centers named 1 and 6:

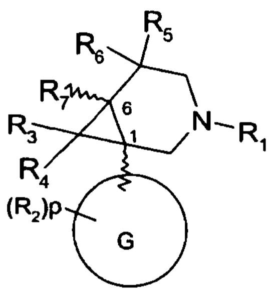

(I)"

wherein $R_1$, $R_2$, $R_3$, $R_4$, $R_5$, $R_6$, $R_7$, p and G are defined as above for compounds of formula (I).

In the context of the present invention, the representation shown above in compounds of formula (I)" for the two bonds near the cyclopropyl moiety bearing groups G and $R_7$ indicate a cis stereoisomer, which has a single but unknown absolute configuration at stereogenic centers named 1 and 6.

It is intended in the context of the present invention that stereochemical isomers of formula (I)" are enriched in one configuration at centers named 1 and 6. In one embodiment, the isomers correspond to at least 90% e.e. (enantiomeric excess). In another embodiment the isomers correspond to at least 95% e.e. In another embodiment the isomers correspond to at least 99% e.e.

From now on throughout the document, the symbol " (double prime) is used to identify stereochemical isomers of the compounds of the invention having "cis" disposition for the two bonds near the cyclopropyl moiety bearing groups G and $R_7$ and indicated with the representation shown above for compounds of formula (I)", those stereoisomers having a single but unknown absolute configuration at stereogenic centers named 1 and 6.

The absolute configuration of the optical isomers of compounds of the present invention may be assigned using conventional techniques well known in the art [including for example X-Ray analysis and VCD (vibrational circular dichroism) analysis].

In one embodiment of the present invention compounds of formula (IA) are provided that correspond to stereochemical isomers of compounds of formula (I)', having the configuration shown in the picture below at stereogenic centers at position named 1 and 6:

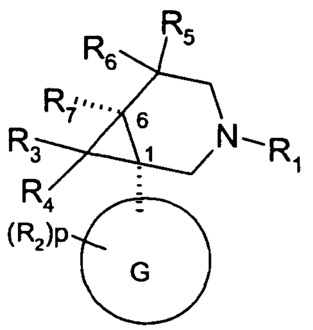

(IA)

wherein $R_1$, $R_2$, $R_3$, $R_4$, $R_5$, $R_6$, $R_7$, p and G are defined as above for compounds of formula (I), or pharmaceutically acceptable salts, or solvates thereof.

It is intended in the context of the present invention that stereochemical isomers of formula (IA) are enriched in one configuration at stereogenic centers named 1 and 6. In one embodiment, the isomers correspond in one embodiment to at least 90% e.e. (enantiomeric excess). In another embodiment the isomers correspond to at least 95% e.e. In another embodiment the isomers correspond to at least 99% e.e.

From now on throughout the document, the suffix "A" in brackets is used to identify stereochemical isomers of compounds of the invention having the configuration shown above for compounds of formula (IA) at stereogenic centers at positions named 1 and 6.

In another embodiment of the present invention compounds of formula (IB) are provided that correspond to stereochemical isomers of compounds of formula (I)', having the configuration shown in the picture below at stereogenic centers at position named 1 and 6:

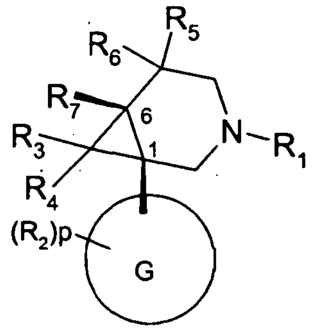

(IB)

wherein $R_1$, $R_2$, $R_3$, $R_4$, $R_5$, $R_6$, $R_7$, p and G are defined as above for compounds of formula (I), or or pharmaceutically acceptable salts or solvates thereof.

It is intended in the context of the present invention that stereochemical isomers of formula (IB) are enriched in one configuration at centers named 1 and 6. In one embodiment, the isomers correspond in one embodiment to at least 90% e.e. (enantiomeric excess). In another embodiment the isomers correspond to at least 95% e.e. In another embodiment the isomers correspond to at least 99% e.e.

From now on throughout the document, the suffix "B" in brackets is used to identify stereochemical isomers of compounds of the invention having the configuration shown above for compounds of formula (IB) at stereogenic centers at positions named 1 and 6.

The term "$C_{1-4}$alkyl" refers to an alkyl group having from one to four carbon atoms, in all isomeric forms, such as methyl, ethyl, propyl, isopropyl, butyl, isobutyl, sec-butyl and tert-butyl.

The term '$C_3$-$C_6$ cycloalkyl group' as used herein means a non aromatic monocyclic hydrocarbon ring of 3 to 6 carbon atom such as, for example, cyclopropyl, cyclobutyl, cyclopentyl, or cyclohexyl; while unsaturated cycloalkyls include cyclopentenyl and cyclohexenyl.

The term '$C_{3-6}$cydoalkyl$C_{1-3}$alkyl' as used herein means an alkyl having from one to three carbon atoms wherein one hydrogen atom is replaced with a $C_3$-$C_6$ cycloalkyl group as above defined, for example methylcyclopropane.

The term "$C_{1-4}$alkoxy" refers to a linear chain or branched chain alkoxy (or "alkyloxy") group having from one to four carbon atoms, such as methoxy, ethoxy, propoxy, isopropoxy, butoxy, isobutoxy, sec-butoxy and tert-butoxy.

The term '$C_{1-4}$ alkanoyl group' as used herein may be a linear or a branched chain alkanoyl group, for example acetyl, ethylcarbonyl, n-propylcarbonyl, i-propyl carbonyl, n-butylcarbonyl or t-butylcarbonyl.

The term 'halo $C_{1-4}$ alkyl' as used herein means an alkyl group having one or more carbon atoms and wherein at least one hydrogen atom is replaced with halogen, preferably fluorine, such as for example a trifluoromethyl group.

The term 'halo $C_{1-4}$ alkoxy group' as used herein may be a $C_{1-4}$ alkoxy group as defined before substituted with at least one halogen, such as $OCH_2CF_3$, $OCHF_2$, or $OCF_3$.

The term 'halo $C_{1-2}$ alkyl group' as used herein may be a $C_{1-2}$ alkyl group as defined before substituted with at least one halogen, preferably fluorine, such as $-CH_2CF_3$, $-CHF_2$, or $-CF_3$.

The term "$SF_5$" refers to pentafluorosulfanyl.

The term "halogen" and its abbreviation "halo" refer to fluorine (F), chlorine (Cl), bromine (Br) or iodine (I). Where the term "halo" is used before another group, it indicates that the group is substituted by one or more halogen atoms.

The term '5,6-membered monocyclic heteroaryl' as used herein means an aromatic monocyclic heterocycle ring of 5 or 6 members and having at least one heteroatom selected from nitrogen, oxygen and sulfur, and containing at least 1 carbon atom. Representative 5, 6 membered monocyclic heteroaryl groups include: furyl, thiophenyl, pyrrolyl, pyridyl, oxazolyl, isooxazolyl, pyrazolyl, imidazolyl, thiazolyl, isothiazolyl, pyridazinyl, pyrimidinyl, pyrazinyl, triazinyl, triazolyl and tetrazolyl.

The term '8, 11-membered bicyclic heteroaryl' as used herein means an aromatic bicyclic heterocycle ring of 8 to 11 members and having at least one heteroatom selected from nitrogen, oxygen and sulfur, and containing at least 1 carbon atom.

Representative 8, to 11 membered bicyclic heteroaryl groups include: benzofuranyl, benzothiophenyl, indolyl, isoindolyl, azaindolyl, quinolinyl, isoquinolinyl, benzoxazolyl, benzimidazolyl, benzothiazolyl, quinazolinyl and phthalazinyl.

The term 5-6 membered heterocycle means a 5-6 monocyclic heterocyclic ring which is either saturated, unsaturated or aromatic, and which contains from 1 to 4 heteroatoms independently selected from nitrogen, oxygen and sulfur, and wherein the nitrogen and sulfur heteroatoms may be optionally oxidized, and the nitrogen heteroatom may be optionally quatemized. Heterocycles include heteroaryl groups as defined above. The heterocycle may be attached via any heteroatom or carbon atom. Thus, the term includes morpholinyl, pyridinyl, pyrazinyl, pyrazolyl, thiazolyl, triazolyl,

imidazolyl, oxadiazolyl, oxazolyl, isoxazolyl, pyrrolidinonyl, pyrrolidinyl, piperidinyl, hydantoinyl, valerolactamyl, oxiranyl, oxetanyl, tetrahydrofuranyl, tetrahydropyranyl, tetrahydropyridinyl, tetrahydropyrimidinyl, tetrahydrothiophenyl, tetrahydrothiopyranyl.

Any of these groups may be attached to the rest of the molecule at any suitable position.

In one embodiment, $R_1$ is hydrogen or $C_{1-4}$ alkyl (for example methyl). In another embodiment, $R_1$ is hydrogen.

In one embodiment, G is a phenyl group.

In one embodiment, $R_3$ is hydrogen or a group X. In another embodiment, $R_3$ is hydrogen. In a further embodiment, $R_3$ is a group X.

In one embodiment, $R_4$ is hydrogen.

In one embodiment, $R_5$ is hydrogen.

In one embodiment, $R_6$ is hydrogen.

In one embodiment, $R_7$ is hydrogen or a group X, $X_1$ or $X_2$. In another embodiment, $R_7$ is hydrogen. In a further embodiment, $R_7$ is a group X.

In one embodiment, n is 1 or 2. In another embodiment, n is 1.

In one embodiment, $R_{10}$ is hydrogen , $C_{1-4}$alkyl, $C_{3-6}$cycloalkyl or $C_{3-6}$cycloalkyl$C_{1-3}$alkyl. In another embodiment, $R_{10}$ is hydrogen or $C_{1-4}$alkyl. In a still further embodiment, $R_{10}$ is $C_{1-4}$alkyl (for example methyl or ethyl).

In one embodiment, $R_2$ is halogen ( for example chlorine) or halo$C_{1-4}$alkyl ( for example trifluoromethyl). In a further embodiment, $R_2$ is chlorine.

In one embodiment, p is 0, 1 or 2. In another embodiment p is 1 or 2. In a further embodiment p is 2.

In one embodiment, a compound of formula (IC) or a salt thereof is provided, wherein G is a phenyl group and $R_1$, $R_2$, p and $R_7$ are as defined for formula (I):

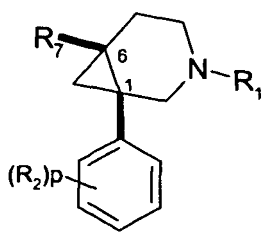

(IC)

In Formula (IC), in one embodiment, $R_1$ is hydrogen or $C_{1-4}$alkyl (for example methyl), $R_2$ is halogen (for example chlorine), p is 0, 1 or 2 and $R_7$ is hydrogen or a group X, $X_1$ or $X_2$. In Formula (IC), in a further embodiment, $R_1$ is hydrogen, $R_2$ is halogen (for example chlorine), p is 2, and $R_7$ is hydrogen or a group X, $X_1$ or $X_2$.

In one embodiment, a compound of formula (ID) or a salt thereof is provided, wherein $R_7$ is a group X and $R_2$, $R_{10}$, p and n are as defined for formula (I):

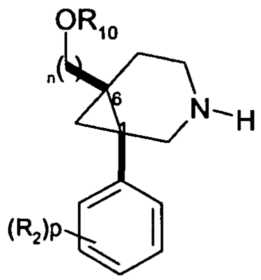

(ID)

In Formula (ID), in one embodiment, $R_2$ is halogen (for example chlorine), p is 0, 1 or 2 and $R_{10}$ is hydrogen , $C_{1-4}$alkyl, $C_{3-6}$cycloalkyl or $C_{3-6}$cycloalkyl$C_{1-3}$alkyl.

In Formula (ID), in a further embodiment, $R_2$ is halogen (for example chlorine), p is 2, n is 1 and $R_{10}$ is hydrogen or $C_{1-4}$alkyl (for example methyl).

In one embodiment, a compound of formula (IE) or a salt thereof is provided, wherein G is a phenyl group and $R_1$, $R_2$, $R_3$ and p are as defined for formula (I):

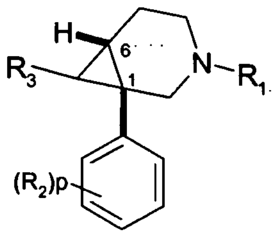

(IE)

In Formula (IE), in one embodiment, $R_1$ is hydrogen or $C_{1-4}$alkyl (for example methyl), $R_2$ is halogen (for example chlorine), p is 0, 1 or 2 and $R_3$ is a group X or $X_1$.

In Formula (IE), in a further embodiment, $R_1$ is hydrogen, $R_2$ is halogen (for example chlorine), p is 2, and $R_3$ is a group X.

In one embodiment, compounds of formula (IC), (ID), (IF) and (IE) as above defined, having a single but unknown configuration at stereogenic centers at position named 1 and 6, are provided . Those compounds are named (IC)", (ID)", (IF)" and (IE)".

In another embodiment, compounds of formula (IC), (ID), (IF) and (IE) as above defined, having the configuration shown above for compounds of formula (IA) at stereogenic centers at position named 1 and 6, are provided . Those compounds are named (ICA), (IDA), (IFA) and (IEA).

In a further embodiment, compounds of formula (IC), (ID), (IF) and (IE) as above defined, having the configuration shown above for compounds of formula (IB) at stereogenic centers at position named 1 and 6, are provided . Those compounds are named (ICB), (IDB), (IFB) and (IEB).

Certain groups/substituents included in the present invention maybe present as isomers. The present invention includes within its scope all such isomers, including racemates, enantiomers, tautomers and mixtures thereof.

Certain groups in compounds of formula (I) or in intermediates used to prepare them, may exist in one or more tautomeric forms. The present invention includes within its scope all such tautomeric forms, including mixtures.

As used herein, the term "salt" refers to any salt of a compound according to the present invention prepared from an inorganic or organic acid or base, quaternary ammonium salts and internally formed salts and also includes pharmaceutically acceptable salts. Pharmaceutically acceptable salts are particularly suitable for medical applications because of their greater aqueous solubility relative to the parent compounds. Such salts must clearly have a physiologically acceptable anion or cation.

Certain of the compounds of the invention may form acid or base addition salts with one or more equivalents of the acid or of the base. The present invention includes within its scope all possible stoichiometric and non-stoichiometric forms.

Pharmaceutically acceptable salts may also be prepared from other salts, including other pharmaceutically acceptable salts, of the compound of formula (I) using conventional methods.

Suitably pharmaceutically acceptable salts of the compounds of the present invention include acid addition salts formed with inorganic acids such as hydrochloric, hydrobromic, hydroiodic, phosphoric, metaphosphoric, nitric and sulfuric acids, and with organic acids, such as tartaric, acetic, trifluoroacetic, citric, malic, lactic, fumaric, benzoic, naphtoic, formic, propionic, glycolic, gluconic, maleic, succinic, camphorsulfuric, isothionic, mucic, gentisic, isonicotinic, saccharic, glucuronic, furoic, glutamic, ascorbic, anthranilic, salicylic, phenylacetic, mandelic, embonic (pamoic), methanesulfonic, ethanesulfonic, pantothenic, stearic, sulfinilic, alginic, galacturonic and arylsulfonic, for example benzenesulfonic and p-toluenesulfonic, acids; base addition salts formed with alkali metals and alkaline earth metals and organic bases such as N,N-dibenzylethylenediamine, chloroprocaine, choline, diethanolamine, ethylenediamine, meglumaine (N-methylglucamine), lysine and procaine; and internally formed salts.

Those skilled in the art of organic chemistry will appreciate that many organic compounds can form complexes with solvents in which they are reacted or from which they are precipitated or crystallized. These complexes are known as "solvates". For example, a complex with water is known as a "hydrate". Solvates of the compounds of the invention are within the scope of the invention. The compounds of formula (I) may readily be isolated in association with solvent molecules by crystallisation or evaporation of an appropriate solvent to give the corresponding solvates.

Hereinafter, compounds of formula (I) and their pharmaceutically acceptable salts or solvates defined in any aspect of the invention (except intermediate compounds in chemical processes) are referred to as "compounds of the invention".

Furthermore, some of the crystalline forms of the compounds of the present invention, may exist as polymorphs, which are included in the present invention.

Those skilled in the art will appreciate that in the preparation of the compounds of the invention, it may be

necessary and/or desirable to protect one or more sensitive groups in the molecule to prevent undesirable side reactions. Suitable protecting groups for use according to the present invention are well known to those skilled in the art and may be used in a conventional manner. See, for example, "Protective groups in organic synthesis" by T.W. Greene and P.G.M. Wuts (John Wiley & sons 1991) or "Protecting Groups" by P.J. Kocienski (Georg Thieme Verlag 1994). Examples of suitable amino protecting groups include acyl type protecting groups (e.g: formyl, trifluoroacetyl, acetyl), aromatic urethane type protecting groups (e.g. benzyloxycarbonyl (Cbz) and substituted Cbz), aliphatic urethane protecting groups (e.g. 9-fluorenylmethoxycarbonyl (Fmoc), t-butyloxycarbonyl (Boc), isopropyloxycarbonyl, cyclohexyloxycarbonyl) and alkyl type protecting groups (e.g. benzyl, trityl, chlorotrityl). Examples of suitable oxygen protecting groups may include for example alky silyl groups, such as trimethylsilyl or tertbutyldimethylsilyl; alkyl ethers such as tetrahydropyranyl or tert-butyl; or esters such as acetate.

The present invention also includes isotopically-labelled compounds, which are identical to those recited in formula (I) and following, but for the fact that one or more atoms are replaced by an atom having an atomic mass or mass number different from the atomic mass or mass number usually found in nature. Examples of isotopes that can be incorporated into compounds of the invention and pharmaceutically acceptable salts thereof include isotopes of hydrogen, carbon, nitrogen, oxygen, phosphorous, sulphur, fluorine, iodine, and chlorine, such as $^{2}H$, $^{3}H$, $^{11}C$, $^{13}C$, $^{14}C$, $^{15}N$, $^{17}O$, $^{18}O$, $^{31}P$, $^{32}P$, $^{35}S$, $^{18}F$, $^{36}Cl$, $^{123}I$ and $^{125}I$.

Compounds of the present invention and non-pharmaceutically acceptable salts thereof that contain the aforementioned isotopes and/or other isotopes of other atoms are within the scope of the present invention. Isotopically-labelled compounds of the present invention, for example those into which radioactive isotopes such as $^{3}H$, $^{14}C$ are incorporated, are useful in drug and/or substrate tissue distribution assays. Tritiated, i.e., $^{3}H$, and carbon-14, i.e., $^{14}C$, isotopes are particularly preferred for their ease of preparation and detectability. $^{11}C$ and $^{18}F$ isotopes are particularly useful in PET (positron emission tomography), and $^{125}I$ isotopes are particularly useful in SPECT (single photon emission computerized tomography), all useful in brain imaging. Further, substitution with heavier isotopes such as deuterium, i.e., $^{2}H$, can afford certain therapeutic advantages resulting from greater metabolic stability, for example increased *in vivo* half-life or reduced dosage requirements and, hence, may be preferred in some circumstances. Isotopically labelled compounds of the present invention and non-pharmaceutically acceptable salts thereof can generally be prepared by carrying out the procedures disclosed in the Schemes and/or in the Examples below, by substituting a readily available isotopically labelled reagent for a non-isotopically labelled reagent.

In one embodiment, the compounds of the invention are selected from the list consisting of:

(1S,6R/1R,6S)-1-(3,4-dichlorophenyl)-3-azabicyclo[4.1.0]heptane;

and pharmaceutically acceptable salts or solvates thereof.

In another embodiment, the compounds of the invention are selected from the list consisting of:

(1*R*,6*R*/1*S*,6*R*)-1-phenyl-3-azabicyclo[4.1.0]heptane;
(1*R*,6*R or 1S*,6*R*)-1-phenyl-3-azabicyclo[4.1.0]heptane;
(*1S,6R* or 1*R*,6*R*)-1-phenyl-3-azabicyclo[4.1.0]heptane;
(1*R*,6*R*/*1S*, 6*S*)-1-[4-(trifluoromethyl)phenyl]-3-azabicyclo[4.1.0]heptane;
(1*R*,6*R*/*1S*,6*S*)-1-[3-(trifluoromethyl)phenyl]-3-azabicyclo[4.1.0]heptane;

and pharmaceutically acceptable salts or solvates thereof.

In a further embodiment, the compounds of the invention are selected from the list consisting of:

(1S,6R/1R,6S)-1-(3,4-dichlorophenyl)-3-azabicyclo[4.1.0]heptane;
(1S,6R or 1R,6S)-1-(3,4-dichlorophenyl)-3-azabicyclo[4.1.0]heptane;
(1R,6S or 1S,6R)-1-(3,4-dichlorophenyl)-3-azabicyclo[4.1.0]heptane;
(1*R*,6*R*)/(1S,6S)-1-(3,4-dichlorophenyl)-6-[(methyloxy)methyl]-3-azabicyclo[4.1.0]heptane;
(1*R*,6*R* or 1S,6S)-1-(3,4-dichlorophenyl)-6-[(methyloxy)methyl]-3-azabicyclo[4.1.0]heptane;
(1S,6S or 1*R*,6*R*)-1-(3,4-dichlorophenyl)-6-[(methyloxy)methyl]-3-azabicyclo[4.1.0]heptane;
(1*R*,7*S*/1*S*,7*R*)-1-(3,4-dichlorophenyl)-7-[(methyloxy)methyl]-3-azabicyclo[4.1.0]heptane; and pharmaceutically acceptable salts or solvates thereof.

The present invention also provides a process for preparing a compound of formula (I) or a salt thereof as defined above.

Compounds of the invention may be prepared according to the following synthetic schemes, wherein the broadest scope has been defined using formula (I) as basis.

Throughout the specification, general formulae are designated by Roman numerals (I), (II), (III), (IV) etc. Subsets

of these general formulae are defined as (Ia), (Ib), (Ic) etc .... (IVa), (IVb), (IVc) etc.

In the following reaction schemes, unless otherwise stated $R_1$ to $R_{11}$, X, $X_1$, $X_2$, $X_3$, G, p and n are as for compounds of formula (I).

Compounds of formula (Ib), i.e. compounds of formula (I) wherein $R_1 = C_{1-4}$ alkyl, may be obtained by compounds of formula (Ia), i.e. compounds of formula (I) wherein $R_1$ = H, following standard alkylation procedures, e. g. using a RX alkylating agent (R = $C_{1-4}$alkyl, X = halogen), such as $CH_3I$, a trialkylamine, such as TEA, in DCM, at temperature between 0 °C and room temperature, according to Scheme 1.

Scheme 1

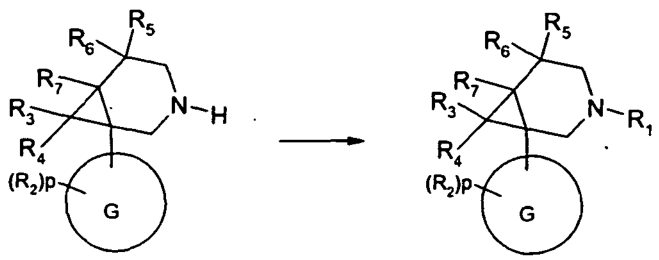

(Ia) (Ib)

Compounds of formula (Ia) may be obtained, according to Scheme 2, through reduction of the mixture of compounds (III) and (II) followed by chromatographic separation of the different regioisomers obtained.

Scheme 2.

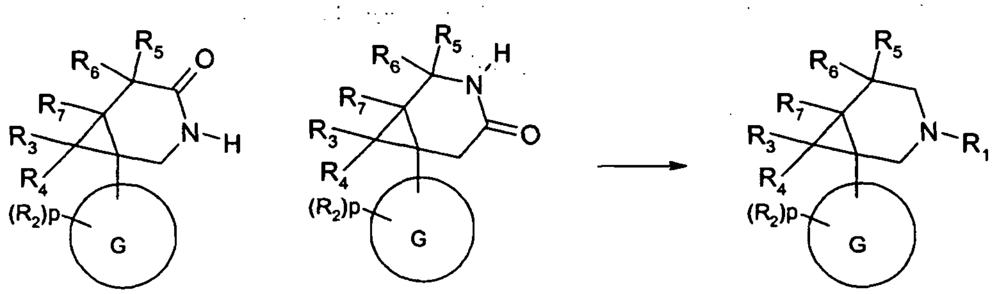

(III) (II) (Ia)

In particular, the reduction reaction may be performed using borane in THF at refluxing temperature.

Compounds of formula (III) and (II) may be obtained by compounds of formula (IV) via Beckmann rearrangement using tosyl chloride in acetone from room temperature to reflux according to Scheme 3.

Scheme 3

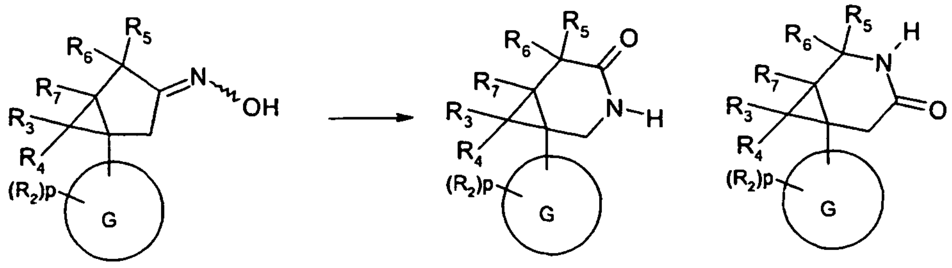

(IV) (III) (II)

Compounds of formula (IV) may be obtained from compounds of formula (V) according to Scheme 4 using hydroxylamine monohydrate in ethanol at room temperature.

Scheme 4

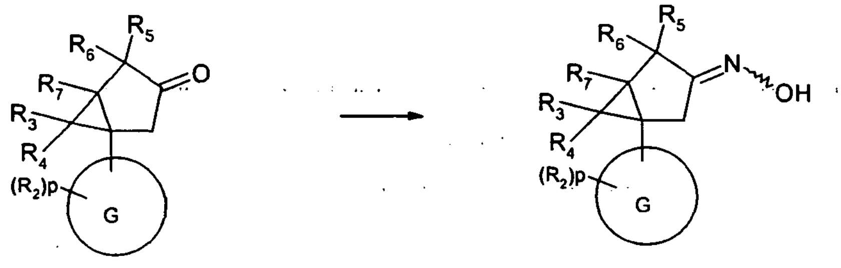

(V) (IV)

Compounds of formula (V) may be obtained from compounds of formula (VI), wherein $R_2$ and p are defined as for formula (I), according to Scheme 5 by rearrangement of the appropriate propargylic aldehyde in accordance to the method described in J. Am. Chem. Soc. 2004, 126, 8654, after reaction with the allylic derivative (VII) where M can be $SiMe_2Cl$ or MgBr and $R_3$, $R_4$ and $R_7$ are defined as for formula (I).

Scheme 5

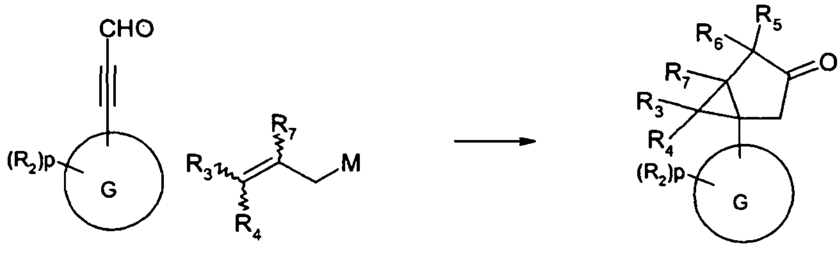

(VI) (VII) (V)

Compounds of formula (VI) may be obtained by oxidation with Dess-Martin periodinane in DCM at room temperature from the appropriate alcohol (VIII), wherein $R_2$ and p are defined as for formula (I), according to Scheme 6.

Scheme 6

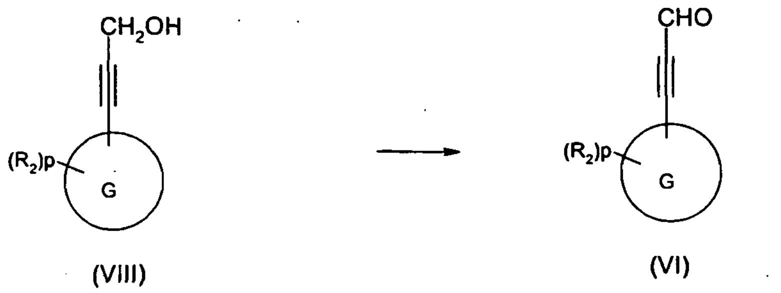

Compounds of formula (VIII) may be obtained according to the method described in JOC, 2005, 70, 4043, from propargyl alcohol and the appropriate lodo arene derivative (IX) according to Scheme 7.

Scheme 7

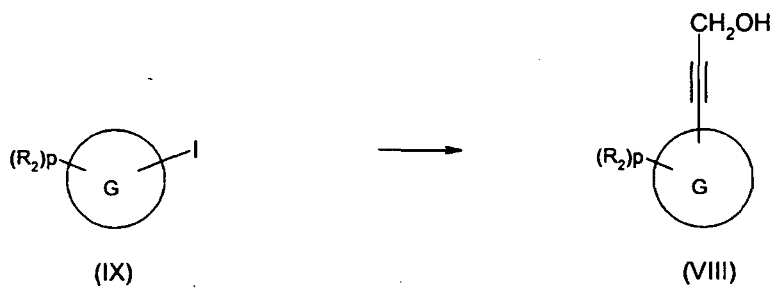

Compounds of formula (Ic), i.e. compounds of formula (I) wherein $R_7$ = $CH_2OH$, may be obtained from compounds of formula (XVI), wherein Pg is a suitable N-protecting group (typically Cbz or Boc), through deprotection of N-Pg group, (such as for Cbz using 6N hydrochloridric acid in dioxane at reflux temperature or for BOC using TFA in DCM at temperature between 0 °C and room temperature) according to Scheme 8.

Scheme 8

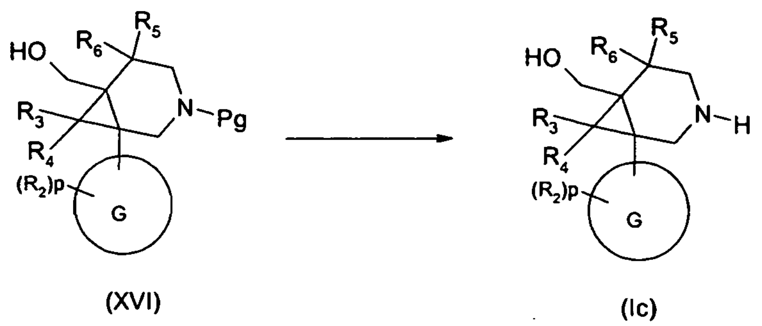

Compounds of formula (XVI) may be obtained from compounds of formula (X), wherein $R_2$, $R_5$, $R_6$ are defined

as above and Pg is a suitable N-protecting group, according to Scheme 9, through the standard Simmons-Smith cyclopropanation procedure (e.g. using $ZnEt_2$, $CH_2I_2$ in DCM).

Scheme 9

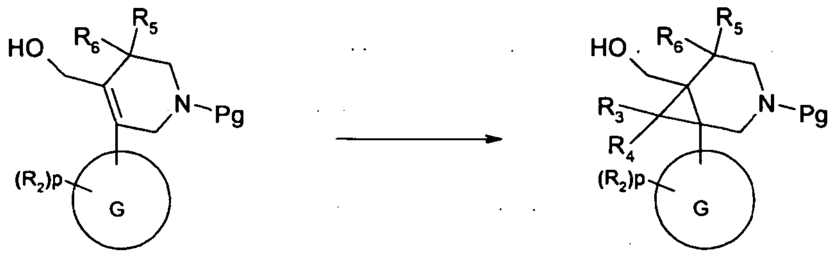

(X) (XVI)

Compounds of formula (X) may be obtained from compounds of formula (XI), according to Scheme 10, using reducing agents, such as $LiAlH_4$, in aprotic solvent, e. g. diethyl ether or THF, at temperature between -40 and -10 °C.

Scheme 10

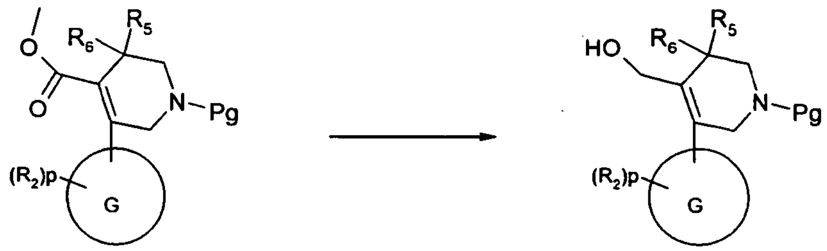

(XI) (X)

Compounds of formula (XI) may be obtained from compounds of formula (XII), according to Scheme 11, through a suitable protecting agent, such as reaction with Cbz-chloride or Boc anhydride, using TEA in DCM at temperature between 0 °C and room temperature.

Scheme 11

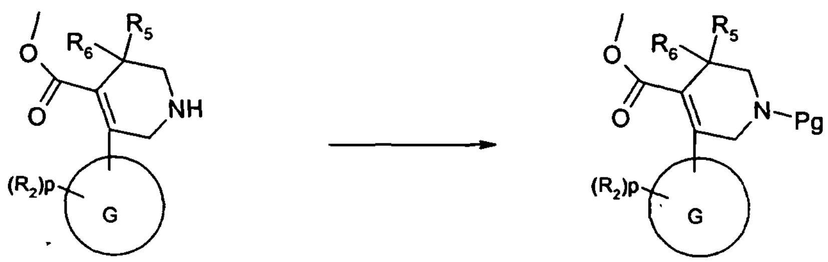

(XII) (XI)

Compounds of formula (XII) may be obtained from compounds of formula (XIII), according to Scheme 12, through reaction with 1-chloroethyl chloroformate in DCE and MeOH.

Scheme 12

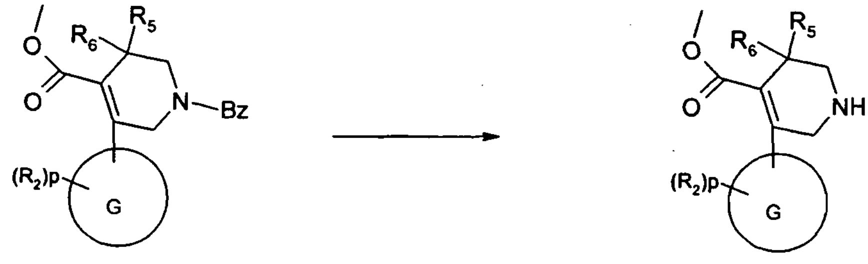

(XIII) (XII)

Compounds of formula (XIII) may be obtained from compounds of formula (XIV), according to Scheme 13, following the standard Suzuky coupling procedure using the appropriate aryl boronic acids or boronate esters, $Pd(PPh_3)_4$ and a base, e. g. $Na_2CO_3$ in a mixture of solvent e. g. toluene, ethanol and water at 80 °C.

Scheme 13

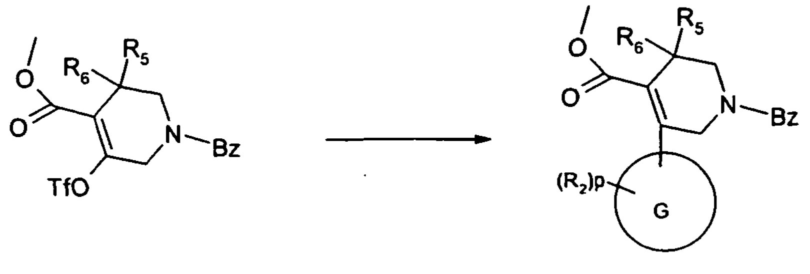

(XIV) (XIII)

Compounds of formula (XIV) may be obtained according to Scheme 14 from compounds of formula (XV), by reaction with a base (eg sodium hydride), then with a triflating agent, such as N-phenyltrifluoromethanesulfonimide, in an aprotic solvent (eg DMF), at temperature between 0 °C and room temperature.

Scheme 14

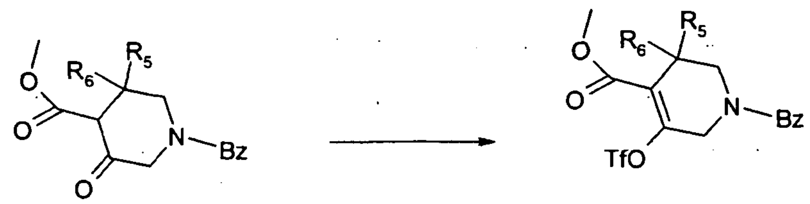

(XV) (XIV)

When a specific enantiomer or diastereoisomer of a compound of formula (I)' or salts thereof, is required, this may be obtained for example by resolution of a corresponding enantiomeric or diastereoisomeric mixture using conventional methods.

Thus, for example, specific enantiomers or diastereoisomers of the compounds may be obtained from the corresponding enantiomeric or diastereoisomeric mixture using chiral chromatographic methods such as for example chiral HPLC (for reference procedure see for example separation of E1 Enant 1 and E1 Enant 2).

Alternatively, specific enantiomers or diastereoisomers of the compounds may be obtained from the corresponding enantiomeric or diastereoisomeric mixture using chiral crystallization methods such as precipitation with chiral acids.

Furthermore a specific enantiomer or diastereoisomer of a compound of the invention may be synthesised from the appropriate optically active intermediate using any of the general processes described herein.
Alternatively, a specific enantiomer or diastereoisomer of a compound the invention may be synthesised from the appropriate stereochemically enriched intermediate using any of the general processes described herein and by combining it with any of the conventional resolution methods above described.

Optically active intermediates or stereochemically enriched intermediates, may be generated by resolution of a corresponding enantiomeric or diastereosiomeric mixtures using conventional methods, or by performance of stereoselective reactions or by combining different resolution techniques.

Also specific enantiomers or diastereoisomers of the compounds may be obtained by combining conventional methods above described.

The compounds of the present invention are useful in the treatment of disorders or diseases responsive to the monoamine neurotransmitter re-uptake inhibiting activity of the compounds. This activity of the compounds of the invention may make them useful in the treatment of Parkinsonism, depression, eating disorders, sleep disorders, substance related disorders, attention-deficit hyperactivity disorders, anxiety disorders, cognition impairment, sexual dysfunctions, obsessive compulsive spectrum disorders, Gilles de la Tourettes disease and senile dementia, as well as other disorders sensitive to the monoamine neurotransmitter re-uptake-inhibiting activity of the compounds.

Within the context of the present invention, the terms describing some indications used herein are classified in the Diagnostic and Statistical Manual of Mental Disorders, 4th Edition, published by the American Psychiatric Association (DSM-IV) and/or the International Classification of Diseases, 10th Edition (ICD-10). The various subtypes of the disorders mentioned herein are contemplated as part of the present invention. Numbers in brackets after the listed diseases below refer to the classification code in DSM-IV.

The term "depression" includes:

Depression and mood disorders including Major Depressive Episode, Manic Episode, Mixed Episode and Hypomanic Episode; Depressive Disorders including Major Depressive Disorder, Dysthymic Disorder (300.4), Depressive Disorder Not Otherwise Specified (311); Other Mood Disorders including Mood Disorder Due to a General Medical Condition (293.83) which includes the subtypes With Depressive Features, With Major Depressive-like Episode, With Manic Features and With Mixed Features), Substance-Induced Mood Disorder (including the subtypes With Depressive Features, With Manic Features and With Mixed Features) and Mood Disorder Not Otherwise Specified (296.90): Bipolar Disorders including Bipolar I Disorder, Bipolar II Disorder (Recurrent Major Depressive Episodes with Hypomanic Episodes) (296.89), Cyclothymic Disorder (301.13) and Bipolar Disorder Not Otherwise Specified (296.80);

The term "anxiety disorders" includes:

Anxiety disorders including Panic Attack; Panic Disorder including Panic Disorder without Agoraphobia (300.01) and Panic Disorder with Agoraphobia (300.21); Agoraphobia; Agoraphobia Without History of Panic Disorder (300.22), Specific Phobia (300.29, formerly Simple Phobia) including the subtypes Animal Type, Natural Environment Type, Blood-Injection-Injury Type, Situational Type and Other Type), Social Phobia (Social Anxiety Disorder, 300.23), Obsessive-Compulsive Disorder (300.3), Posttraumatic Stress Disorder (309.81), Acute Stress Disorder (308.3), Generalized Anxiety Disorder (300.02), Anxiety Disorder Due to a General Medical Condition (293.84), Substance-Induced Anxiety Disorder, Separation Anxiety Disorder (309.21), Adjustment Disorders with Anxiety (309.24) and Anxiety Disorder Not Otherwise Specified (300.00):

The term "substance related disorder" includes:

Substance-related disorders including Substance Use Disorders such as Substance Dependence, Substance Craving and Substance Abuse; Substance-Induced Disorders such as Substance Intoxication, Substance Withdrawal, Substance-Induced Delirium, Substance-Induced Persisting Dementia, Substance-Induced Persisting Amnestic

Disorder, Substance-Induced Psychotic Disorder, Substance-Induced Mood Disorder, Substance-Induced Anxiety Disorder, Substance-Induced Sexual Dysfunction, Substance-Induced Sleep Disorder and Hallucinogen Persisting Perception Disorder (Flashbacks); Alcohol-Related Disorders such as Alcohol Dependence (303.90), Alcohol Abuse (305.00), Alcohol Intoxication (303.00), Alcohol Withdrawal (291.81), Alcohol Intoxication Delirium,

Alcohol Withdrawal Delirium, Alcohol-Induced Persisting Dementia, Alcohol-Induced Persisting Amnestic Disorder, Alcohol-Induced Psychotic Disorder, Alcohol-Induced Mood Disorder, Alcohol-Induced Anxiety Disorder, Alcohol-Induced Sexual Dysfunction, Alcohol-Induced Sleep Disorder and Alcohol-Related Disorder Not Otherwise Specified (291.9); Amphetamine (or Amphetamine-Like)-Related Disorders such as Amphetamine Dependence (304.40), Amphetamine Abuse (305.70), Amphetamine Intoxication (292.89), Amphetamine Withdrawal (292.0), Amphetamine Intoxication Delirium, Amphetamine Induced Psychotic Disorder, Amphetamine-Induced Mood Disorder, Amphetamine-Induced Anxiety Disorder, Amphetamine-Induced Sexual Dysfunction, Amphetamine-Induced Sleep Disorder and Amphetamine-Related Disorder Not Otherwise Specified (292.9); Caffeine Related Disorders such as Caffeine Intoxication (305.90), Caffeine-Induced Anxiety Disorder, Caffeine-Induced Sleep Disorder and Caffeine-Related Disorder Not Otherwise Specified (292.9); Cannabis-Related Disorders such as Cannabis Dependence (304.30), Cannabis Abuse (305.20), Cannabis Intoxication (292.89), Cannabis Intoxication Delirium, Cannabis-Induced Psychotic Disorder, Cannabis-Induced Anxiety Disorder and Cannabis-Related Disorder Not Otherwise Specified (292.9); Cocaine-Related Disorders such as Cocaine Dependence (304.20), Cocaine Abuse (305.60), Cocaine Intoxication (292.89), Cocaine Withdrawal (292.0), Cocaine Intoxication Delirium, Cocaine-Induced Psychotic Disorder, Cocaine-Induced Mood Disorder, Cocaine-Induced Anxiety Disorder, Cocaine-Induced Sexual Dysfunction, Cocaine-Induced Sleep Disorder and Cocaine-Related Disorder Not Otherwise Specified (292.9); Hallucinogen-Related Disorders such as Hallucinogen Dependence (304.50), Hallucinogen Abuse (305.30), Hallucinogen Intoxication (292.89), Hallucinogen Persisting Perception Disorder (Flashbacks) (292.89), Hallucinogen Intoxication Delirium, Hallucinogen-Induced Psychotic Disorder, Hallucinogen-Induced Mood Disorder, Hallucinogen-Induced Anxiety Disorder and Hallucinogen-Related Disorder Not Otherwise Specified (292.9); Inhalant-Related Disorders such as Inhalant Dependence (304.60), Inhalant Abuse (305.90), Inhalant Intoxication (292.89), Inhalant Intoxication Delirium, Inhalant-Induced Persisting Dementia, Inhalant-Induced Psychotic Disorder, Inhalant-Induced Mood Disorder, Inhalant-Induced Anxiety Disorder and Inhalant-Related Disorder Not Otherwise Specified (292.9); Nicotine-Related Disorders such as Nicotine Dependence (305.1), Nicotine Withdrawal (292.0) and Nicotine-Related Disorder Not Otherwise Specified (292.9); Opioid-Related Disorders such as Opioid Dependence (304.00), Opioid Abuse (305.50), Opioid Intoxication (292.89), Opioid Withdrawal (292.0), Opioid Intoxication Delirium, Opioid-Induced Psychotic Disorder, Opioid-Induced Mood Disorder, Opioid-Induced Sexual Dysfunction, Opioid-Induced Sleep Disorder and Opioid-Related Disorder Not Otherwise Specified (292.9); Phencyclidine (or Phencyclidine-Like)-Related Disorders such as Phencyclidine Dependence (304.60), Phencyclidine Abuse (305.90), Phencyclidine Intoxication (292.89), Phencyclidine Intoxication Delirium, Phencyclidine-Induced Psychotic Disorder, Phencyclidine-Induced Mood Disorder, Phencyclidine-Induced Anxiety Disorder and Phencyclidine-Related Disorder Not Otherwise Specified (292.9); Sedative-, Hypnotic-, or Anxiolytic-Related Disorders such as Sedative, Hypnotic, or Anxiolytic Dependence (304.10), Sedative, Hypnotic, or Anxiolytic Abuse (305.40), Sedative, Hypnotic, or Anxiolytic Intoxication (292.89), Sedative, Hypnotic, or Anxiolytic Withdrawal (292.0), Sedative, Hypnotic, or Anxiolytic Intoxication Delirium, Sedative, Hypnotic, or Anxiolytic Withdrawal Delirium, Sedative-, Hypnotic-, or Anxiolytic-Persisting Dementia, Sedative-, Hypnotic-, or Anxiolytic-Persisting Amnestic Disorder, Sedative-, Hypnotic-, or Anxiolytic-Induced Psychotic Disorder, Sedative-, Hypnotic-, or Anxiolytic-Induced Mood Disorder, Sedative-, Hypnotic-, or Anxiolytic-Induced Anxiety Disorder Sedative-, Hypnotic-, or Anxiolytic-Induced Sexual Dysfunction, Sedative-, Hypnotic-, or Anxiolytic-Induced Sleep Disorder and Sedative-, Hypnotic-, or Anxiolytic-Related Disorder Not Otherwise Specified (292.9); Polysubstance-Related Disorder such as Polysubstance Dependence (304.80); and Other (or Unknown) Substance-Related Disorders such as Anabolic Steroids, Nitrate Inhalants and Nitrous Oxide;

The term "Sleep disorder" includes:

Sleep disorders including primary sleep disorders such as Dyssomnias such as Primary Insomnia (307.42), Primary Hypersomnia (307.44), Narcolepsy (347), Breathing-Related Sleep Disorders (780.59), Circadian Rhythm Sleep Disorder (307.45) and Dyssomnia Not Otherwise Specified (307.47); primary sleep disorders such as Parasomnias such as Nightmare Disorder (307.47), Sleep Terror Disorder (307.46), Sleepwalking Disorder (307.46) and Parasomnia Not Otherwise Specified (307.47); Sleep Disorders Related to Another Mental Disorder such as Insomnia Related to Another Mental Disorder (307.42) and Hypersomnia Related to Another Mental Disorder (307.44); Sleep Disorder Due to a General Medical Condition; and Substance-Induced Sleep Disorder including the subtypes Insomnia Type, Hypersomnia Type, Parasomnia Type and Mixed Type;

The term "eating disorder" include:

Eating disorders such as Anorexia Nervosa (307.1) including the subtypes Restricting Type and Binge-Eating/Purging Type; Bulimia Nervosa (307.51) including the subtypes Purging Type and Nonpurging Type; Obesity; Compulsive Eating Disorder; Binge Eating Disorder; and Eating Disorder Not Otherwise Specified (307.50):

The term "Attention-Deficit/Hyperactivity Disorder" includes:

Attention-Deficit/Hyperactivity Disorder including the subtypes Attention-Deficit /Hyperactivity Disorder Combined Type (314.01), Attention-Deficit /Hyperactivity Disorder Predominantly Inattentive Type (314.00), Attention-Deficit /Hyperactivity Disorder Hyperactive-Impulse Type (314.01) and Attention-Deficit /Hyperactivity Disorder Not Otherwise Specified (314.9); Hyperkinetic Disorder; Disruptive Behaviour Disorders such as Conduct Disorder including the subtypes childhood-onset type (321.81), Adolescent-Onset Type (312.82) and Unspecified Onset (312.89), Oppositional Defiant Disorder (313.81) and Disruptive Behaviour Disorder Not Otherwise Specified; and Tic Disorders such as Tourette's Disorder (307.23);

The term "Cognition impairment" includes:

Cognition impairment including cognition impairment in other diseases such as schizophrenia, bipolar disorder, depression, other psychiatric disorders and psychotic conditions associated with cognitive impairment, e.g. Alzheimer's disease;

The term "Sexual dysfunctions" includes:

Sexual dysfunctions including Sexual Desire Disorders such as Hypoactive Sexual Desire Disorder (302.71), and Sexual Aversion Disorder (302.79); sexual arousal disorders such as Female Sexual Arousal Disorder (302.72) and Male Erectile Disorder (302.72); orgasmic disorders such as Female Orgasmic Disorder (302.73), Male Orgasmic Disorder (302.74) and Premature Ejaculation (302.75); sexual pain disorder such as Dyspareunia (302.76) and Vaginismus (306.51); Sexual Dysfunction Not Otherwise Specified (302.70); paraphilias such as Exhibitionism (302.4), Fetishism (302.81), Frotteurism (302.89), Pedophilia (302.2), Sexual Masochism (302.83), Sexual Sadism (302.84), Transvestic Fetishism (302.3), Voyeurism (302.82) and Paraphilia Not Otherwise Specified (302.9); gender identity disorders such as Gender Identity Disorder in Children (302.6) and Gender Identity Disorder in Adolescents or Adults (302.85); and Sexual Disorder Not Otherwise Specified (302.9);

The term "Obsessive compulsive spectrum disorder" includes:

Obsessive compulsive spectrum disorder including Obsessive compulsive disorders (300.3), somatoform disorders including body dysmorphic disorder (300.7) and hyperchondriasis (300.7), bulimia nervosa (307.51), anorexia nervosa (307.1), eating disorders not elsewhere classified (307.50) such as binge eating, impulse control disorders not elsewhere classified (including intermitted explosive disorder (312.34), compulsive buying or shopping, repetitive self-mutilation, onychophagia, psychogenic excoriation, kleptomania (312.32), pathological gambling (312.31), trichotillomania (312.39) and internet addiction), paraphilia (302.70) and nonparaphilic sexual addictions, Sydeham's chorea, torticollis, autistic disorders (299.0), compulsive hoarding, and movement disorders, including Tourette's syndrome (307.23).

All of the various forms and sub-forms of the disorders mentioned herein are contemplated as part of the present invention.

In an embodiment, compounds of the invention may be useful as analgesics. For example they may be useful in the treatment of chronic inflammatory pain (e.g. pain associated with rheumatoid arthritis, osteoarthritis, rheumatoid spondylitis, gouty arthritis and juvenile arthritis); musculoskeletal pain; lower back and neck pain; sprains and strains; neuropathic pain; sympathetically maintained pain; myositis; pain associated with cancer and fibromyalgia; pain associated with migraine; pain associated with influenza or other viral infections, such as the common cold; rheumatic fever; pain associated with functional bowel disorders such as non-ulcer dyspepsia, non-cardiac chest pain and irritable bowel syndrome; pain associated with myocardial ischemia; post operative pain; headache; toothache; and dysmenorrhea.

Compounds of the invention may be useful in the treatment of neuropathic pain. Neuropathic pain syndromes can develop following neuronal injury and the resulting pain may persist for months or years, even after the original injury has healed. Neuronal injury may occur in the peripheral nerves, dorsal roots, spinal cord or certain regions in the brain. Neuropathic pain syndromes are traditionally classified according to the disease or event that precipitated them. Neuropathic pain syndromes include: diabetic neuropathy; sciatica; non-specific lower back pain; multiple sclerosis pain; fibromyalgia; HIV-related neuropathy; post-herpetic neuralgia; trigeminal neuralgia; and pain resulting from physical

trauma, amputation, cancer, toxins or chronic inflammatory conditions. These conditions are difficult to treat and although several drugs are known to have limited efficacy, complete pain control is rarely achieved. The symptoms of neuropathic pain are incredibly heterogeneous and are often described as spontaneous shooting and lancinating pain, or ongoing, burning pain. In addition, there is pain associated with normally non-painful sensations such as "pins and needles" (paraesthesias and dysesthesias), increased sensitivity to touch (hyperesthesia), painful sensation following innocuous stimulation (dynamic, static or thermal allodynia), increased sensitivity to noxious stimuli (thermal, cold, mechanical hyperalgesia), continuing pain sensation after removal of the stimulation (hyperpathia) or an absence of or deficit in selective sensory pathways (hypoalgesia).

Compounds of the invention may also be useful in the amelioration of inflammatory disorders, for example in the treatment of skin conditions (e.g. sunburn, burns, eczema, dermatitis, psoriasis); ophthalmic diseases such as glaucoma, retinitis, retinopathies, uveitis and of acute injury to the eye tissue (e.g. conjunctivitis); lung disorders (e.g. asthma, bronchitis, emphysema, allergic rhinitis, respiratory distress syndrome, pigeon fancier's disease, farmer's lung, chronic obstructive pulmonary disease, (COPD); gastrointestinal tract disorders (e.g. aphthous ulcer, Crohn's disease, atopic gastritis, gastritis varialoforme, ulcerative colitis, coeliac disease, regional ileitis, irritable bowel syndrome, inflammatory bowel disease, gastroesophageal reflux disease); other conditions with an inflammatory component such as migraine, multiple sclerosis, myocardial ischemia.

In one embodiment, compounds of the invention are useful in the treatment of depression and anxiety disorders.

In another embodiment, compounds of the invention are useful in the treatment of depression.

"Treatment" includes prophylaxis, where this is appropriate for the relevant condition(s).

In an alternative or further aspect there are provided compounds for use in the treatment of a mammal, including man, in particular in the treatment of disorders or diseases responsive to the monoamine neurotransmitter re-uptake inhibiting activity of the compounds, comprising administration of an effective amount of a compound of the invention.

In one embodiment, the invention provides compounds for use in the treatment of a condition for which inhibition of serotonin (5-HT), dopamine (DA) and norepinephrine (NE), is beneficial, which comprises administering to a mammal (e.g. human) in need thereof an effective amount of a compound of the invention.

In another aspect, the invention provides a compound of the invention for use in therapy.

In one embodiment, the invention provides compounds of the invention for use in the treatment of a condition in a mammal for which inhibition of serotonin (5-HT), dopamine (DA) and norepinephrine (NE) is beneficial.

In one aspect, the invention provides the use of compounds of the invention, for the manufacture of a medicament for the treatment of disorders or diseases responsive to monoamine neurotransmitter re-uptake inhibiting activity.

In one embodiment, the use of compounds of the invention is provided in the manufacture of a medicament for the treatment of a condition in a mammal for which inhibition of serotonin (5-HT), dopamine (DA) and norepinephrine (NE) is beneficial.

The compounds of the invention may also be used in combination with other therapeutic agents. The invention thus provides, in a further aspect, a combination comprising a compound of the invention together with a further therapeutic agent.

The compounds of the invention may be used in combination with the following agents to treat or prevent psychotic disorders: i) antipsychotics; ii) drugs for extrapyramidal side effects, for example anticholinergics (such as benztropine, biperiden, procyclidine and trihexyphenidyl), antihistamines (such as diphenhydramine) and dopaminergics (such as amantadine); iii) antidepressants; iv) anxiolytics; and v) cognitive enhancers for example cholinesterase inhibitors (such as tacrine, donepezil, rivastigmine and galantamine).

The compounds of the invention may be used in combination with antidepressants to treat or prevent depression and mood disorders.

The compounds of the invention may be used in combination with the following agents to treat or prevent bipolar disease: i) mood stabilisers; ii) antipsychotics; and iii) antidepressants.

The compounds of the invention may be used in combination with the following agents to treat or prevent anxiety disorders: i) anxiolytics; and ii) antidepressants.

The compounds of the invention may be used in combination with the following agents to improve nicotine withdrawal and reduce nicotine craving: i) nicotine replacement therapy for example a sublingual formulation of nicotine beta-cyclodextrin and nicotine patches; and ii) bupropion.

The compounds of the invention may be used in combination with the following agents to improve alcohol withdrawal and reduce alcohol craving: i) NMDA receptor antagonists for example acamprosate; ii) GABA receptor agonists for example tetrabamate; and iii) Opioid receptor antagonists for example naltrexone.

The compounds of the invention may be used in combination with the following agents to improve opiate withdrawal and reduce opiate craving: i) opioid mu receptor agonist/opioid kappa receptor antagonist for example buprenorphine; ii) opioid receptor antagonists for example naltrexone; and iii) vasodilatory antihypertensives for example lofexidine.

The compounds of the invention may be used in combination with the following agents to treat or prevent

sleeping disorders: i) benzodiazepines for example temazepam, lormetazepam, estazolam and triazolam; ii) non-benzodiazepine hypnotics for example zolpidem, zopiclone, zaleplon and indiplon; iii) barbiturates for example aprobarbital, butabarbital, pentobarbital, secobarbita and phenobarbital; iv) antidepressants; v) other sedative-hypnotics for example chloral hydrate and chlormethiazole.

The compounds of the invention may be used in combination with the following agents to treat anorexia: i) appetite stimulants for example cyproheptidine; ii) antidepressants; iii) antipsychotics; iv) zinc; and v) premenstral agents for example pyridoxine and progesterones.

The compounds of the invention may be used in combination with the following agents to treat or prevent bulimia: i) antidepressants; ii) opioid receptor antagonists; iii) antiemetics for example ondansetron; iv) testosterone receptor antagonists for example flutamide; v) mood stabilisers; vi) zinc; and vii) premenstral agents.

The compounds of the invention may be used in combination with the following agents to treat or prevent autism: i) antipsychotics; ii) antidepressants; iii) anxiolytics; and iv) stimulants for example methylphenidate, amphetamine formulations and pemoline.

The compounds of the invention may be used in combination with the following agents to treat or prevent ADHD: i) stimulants for example methylphenidate, amphetamine formulations and pemoline; and ii) non-stimulants for example norepinephrine reuptake inhibitors (such as atomoxetine), alpha 2 adrenoceptor agonists (such as donidine), antidepressants, modafinil, and cholinesterase inhibitors (such as galantamine and donezepil).

The compounds of the invention may be used in combination with the following agents to treat personality disorders: i) antipsychotics; ii) antidepressants; iii) mood stabilisers; and iv) anxiolytics.

The compounds of the invention may be used in combination with the following agents to treat or prevent male sexual dysfunction: i) phosphodiesterase V inhibitors, for example vardenafil and sildenafil; ii) dopamine agonists/ dopamine transport inhibitors for example apomorphine and buproprion; iii) alpha adrenoceptor antagonists for example phentolamine; iv) prostaglandin agonists for example alprostadil; v) testosterone agonists such as testosterone; vi) serotonin transport inhibitors for example serotonin reuptake inhibitors; v) noradrenaline transport inhibitors for example reboxetine and vii) 5-HT1A agonists, for example flibanserine.

The compounds of the invention may be used in combination with the same agents specified for male sexual dysfunction to treat or prevent female sexual dysfunction, and in addition an estrogen agonist such as estradiol.

Antipsychotic drugs include Typical Antipsychotics (for example chlorpromazine, thioridazine, mesoridazine, fluphenazine, perphenazine, prochlorperazine, trifluoperazine, thiothixine, haloperidol, molindone and loxapine); and Atypical Antipsychotics (for example clozapine, olanzapine, risperidone, quetiapine, aripirazole, ziprasidone and amisulpride).

Antidepressant drugs include serotonin reuptake inhibitors (such as citalopram, escitalopram, fluoxetine, paroxetine and sertraline); dual serotonin/noradrenaline reuptake inhibitors (such as venlafaxine, duloxetine and milnacipran); Noradrenaline reuptake inhibitors (such as reboxetine); tricyclic antidepressants (such as amitriptyline, clomipramine, imipramine, maprotiline, nortriptyline and trimipramine); monoamine oxidase inhibitors (such as isocarboxazide, moclobemide, phenelzine and tranylcypromine); and others (such as bupropion, mianserin, mirtazapine, nefazodone and trazodone).

Mood stabiliser drugs include lithium, sodium valproate/valproic acid/divalproex, carbamazepine, lamotrigine, gabapentin, topiramate and tiagabine.

Anxiolytics include benzodiazepines such as alprazolam and lorazepam.

For use in medicine, the compounds of the present invention are usually administered as a standard pharmaceutical composition. The present invention therefore provides in a further aspect a pharmaceutical composition comprising a compound of the invention and a pharmaceutically (i.e physiologically) acceptable carrier. The pharmaceutical composition can be for use in the treatment of any of the conditions described herein.

The compounds of the invention may be administered by any convenient method, for example by oral, parenteral (e.g. intravenous), buccal, sublingual, nasal, rectal or transdermal administration and the pharmaceutical compositions adapted accordingly.

The compounds of the invention which are active when given orally can be formulated as liquids or solids, for example syrups, suspensions or emulsions, tablets, capsules and lozenges.

A liquid formulation will generally consist of a suspension or solution of the compound or salt in a suitable liquid carrier(s) for example an aqueous solvent such as water, ethanol or glycerine, or a non-aqueous solvent, such as polyethylene glycol or an oil. The formulation may also contain a suspending agent, preservative, flavouring or colouring agent.

A composition in the form of a tablet can be prepared using any suitable pharmaceutical carrier(s) routinely used for preparing solid formulations. Examples of such carriers include magnesium stearate, starch, lactose, sucrose and cellulose.

A composition in the form of a capsule can be prepared using routine encapsulation procedures. For example, pellets containing the active ingredient can be prepared using standard carriers and then filled into a hard gelatin capsule;

alternatively, a dispersion or suspension can be prepared using any suitable pharmaceutical carrier(s), for example aqueous gums, celluloses, silicates or oils and the dispersion or suspension then filled into a soft gelatin capsule.

Typical parenteral compositions consist of a solution or suspension of the compound or salt in a sterile aqueous carrier or parenterally acceptable oil, for example polyethylene glycol, polyvinyl pyrrolidone, lecithin, arachis oil or sesame oil. Alternatively, the solution can be lyophilised and then reconstituted with a suitable solvent just prior to administration.

Compositions for nasal administration may conveniently be formulated as aerosols, drops, gels and powders. Aerosol formulations typically comprise a solution or fine suspension of the active substance in a pharmaceutically acceptable aqueous or non-aqueous solvent and are usually presented in single or multidose quantities in sterile form in a sealed container, which can take the form of a cartridge or refill for use with an atomising device. Alternatively the sealed container may be a unitary dispensing device such as a single dose nasal inhaler or an aerosol dispenser fitted with a metering valve which is intended for disposal once the contents of the container have been exhausted. Where the dosage form comprises an aerosol dispenser, it will contain a propellant which can be a compressed gas such as compressed air or an organic propellant such as a fluoro-chlorohydrocarbon. The aerosol dosage forms can also take the form of a pump-atomiser.

Compositions suitable for buccal or sublingual administration include tablets, lozenges and pastilles, wherein the active ingredient is formulated with a carrier such as sugar and acacia, tragacanth, or gelatin and glycerin.

Compositions for rectal administration are conveniently in the form of suppositories containing a conventional suppository base such as cocoa butter.

Compositions suitable for transdermal administration include ointments, gels and patches.

In one embodiment, the composition is in unit dose form such as a tablet, capsule or ampoule.

Each dosage unit for oral administration contains for example from 0.5 to 250 mg (and for parenteral administration contains for example from 0.05 to 25 mg) of a compound of the invention calculated as the free base.

The pharmaceutically acceptable compounds of the invention will normally be administered in a daily dosage regimen (for an adult patient) of, for example, an oral dose of between 1 mg and 500 mg, for example between 1 mg and 400 mg, e.g. between 10 and 250 mg or an intravenous, subcutaneous, or intramuscular dose of between 0.1 mg and 100 mg, for example between 0.1 mg and 50 mg, e.g. between 1 and 25 mg of the compound of the formula (I) or a salt thereof calculated as the free base, the compound being administered 1 to 4 times per day, for example 1 to 2 time a day. In one embodiment, the compound of the invention may be administered once a day.

Suitably the compounds will be administered for a period of continuous therapy, for example for a week or more.

For oral administration a typical dose may be in the range of 1 to 200 mg per day, for example 60 to 200 mg per day.

When a compound of the invention or a pharmaceutically acceptable derivative thereof is used in combination with a second therapeutic agent active against the same disease state the dose of each compound may differ from that when the compound is used alone. Appropriate doses will be readily appreciated by those skilled in the art. It will be appreciated that the amount of a compound of the invention required for use in treatment will vary with the nature of the condition being treated and the age and the condition of the patient and will be ultimately at the discretion of the attendant physician or veterinarian.

The combinations referred to above may conveniently be presented for use in the form of a pharmaceutical formulation and thus pharmaceutical formulations comprising a combination as defined above together with a pharmaceutically acceptable carrier or excipient comprise a further aspect of the invention. The individual components of such combinations may be administered either sequentially or simultaneously in separate or combined pharmaceutical formulations by any convenient route.

The invention is also directed to a novel kit-of-parts that is suitable for use in the treatment of disorders as above defined comprising a first dosage form comprising a compound of the invention and a second dosage form comprising another therapeutic agent, for simultaneous, separate or sequential administration.

When administration is sequential, either the compound of the invention or the second therapeutic agent may be administered first. When administration is simultaneous, the combination may be administered either in the same or different pharmaceutical composition.

When combined in the same formulation it will be appreciated that the two compounds must be stable and compatible with each other and the other components of the formulation. When formulated separately they may be provided in any convenient formulation, conveniently in such manner as are known for such compounds in the art.

## Biological Assays

### Cell biology

**a) Generation of stable LLCPK cell lines expressing hSERT, hNET, and hDAT**

Stable cell line expressing human serotonin transporter (hSERT) may be created by transfecting Lewis Lung Carcinoma Porcine tubule Kidney (LLC-PK1 or LLCPK) cells with hSERT cloned into the mammalian expression vector pCDNA3.1 Hygro(+).
Stable cell line expressing human norepinephrine transporter (hNET) may be created by transfecting LLCPK cells with hNET cloned into the mammalian expression vector pRC/CMV.
Stable cell line expressing human dopamine transporter (hDAT) may be created by transfecting LLCPK cells with hDAT cloned into the mammalian expression vector pDESTCDNA3.1.
One example of reference procedure for transfecting LLCPK cells with hDAT, hSERT and hNET may be found in H. Gu, S.C. Wall and G. Rudnick, J. Biol. Chem. (1994) 269 : 7124-7130.
Each cell line is cultured independently in Dulbecco's modified Eagle's medium (DMEM) containing 10% of Foetal Bovine Serum (FBS) supplemented with 400 μg/ml hygromicin (hSERT) or geneticin at 500 μg/ml (hNET) or at 1000 μg/ml (hDAT). Cells are maintained at 37 °C in a humidified environment containing 5% CO2 in air.

**b) Generation of BacMam viruses for the expression of hSERT, hNET, and hDAT in mammalian cells**

Membranes for the SPA-binding assays are produced by HEK-293F cell infection with BacMam viruses generated for each single human SERT, NET, and DAT transporter. hSERT and hDAT are cloned into pFBMRfA vector whereas hNET is cloned into pFASTBacMam1 vector. The generation and use of BacMam viruses is described in Condreay JP et al, Proc. Natl. Acad. Sci. USA, 1999, 96:127-132 and Hassan NJ et al, Protein Expression and Purification, 47(2): 591-598, 2006.

### Affinity to the human transporters SERT, NET and DAT

The affinities of the compounds of the invention for the human serotonin transporter (SERT), human norepinephrine transporter (NET) and for the human dopamine transporter (DAT) may be determined by one of the assays described below. Such affinity is typically calculated from the $IC_{50}$ obtained in competition experiments as the concentration of a compound necessary to displace 50% of the radiolabeled ligand from the transporter, and is reported as a "$K_i$" value calculated by the following equation:

$$K_i = \frac{IC_{50}}{1 + L / K_D}$$

where L = radioligand and $K_D$ = affinity of radioligand for transporter (Cheng and Prusoff, Biochem. Pharmacol. 22:3099, 1973). In the context of the present invention pKi values (corresponding to the antilogarithm of Ki) are used instead of Ki; pKi results are only estimated to be accurate to about 0.3-0.5.

**a) Filtration binding assay on membranes form hSERT, hNET, and hDAT LLCPK cell lines**

• Membrane preparation

hSERT-LLCPK or hDAT-LLCPK or hNET-LLCPK cell lines are used for the membrane preparations for radioligand binding assays. Each cell line is cultured independently in Dulbecco's modified Eagle's medium (DMEM) containing 10% of Foetal Bovine Serum (FBS) supplemented with 400 μg/ml hygromicin (hSERT) or geneticin at 500 μg/ml (hNET) or at 1000 μg/ml (hDAT). When cells are at 70-80% of confluence, the culture medium is removed and the cells harvested with phosphate buffered saline (PBS) containing 5 mM EDTA. Cell suspension is centrifuged at 900g for 5 minutes at 4 °C. The resultant pellets are re-suspended in 30-50 volumes of Assay Buffer (50mM Tris pH 7.7 containing 120mM NaCl, 5mM KCl, 10μM pargyline and 0.1 % ascorbic acid) and homogenized using a glass-teflon Potter homogeniser and centrifuged at 48000g for 20 minutes at 4 °C. The resultant membrane pellets are re-suspended in the same volume of Assay Buffer, incubated for 20 minutes at 37 °C and centrifuged as before at 48000g. The final protein concentration for each preparation is adjusted to give approximately 480μg protein/ml for hSERT-LLCPK, hDAT-LLCPK

and hNET-LLCPK, as determined by the Bio-Rad Protein Assay kit. Membranes are stored at -80 °C as 1 ml aliquots until required.

• Filtration assay protocol for hSERT, hNET, and hDAT

General references for monoamine transporters filtration binding assay may be: Michael J. Owens, et al, Neurotrasmitter receptor and transporter binding profile of antidepressants and their metabolites, JPET, 283:1305-1322, 1997; Per Allard, Jan O. Marcusson, Svate B. Ross, [3H]WIN-35,428 binding in the human brain, Brain Res., 706 : 347-350, 1996.
The affinity of the compounds of the invention to bind the re-uptake site of SERT may be assessed using [$^3$H]citalopram filtration binding assay performed on hSERT-LLCPK cell membranes. In details, competition binding assay is conducted in deep-well 96 well plate (1ml, NUNC, cod.260252) in a total volume of 400μl, with each concentration in duplicate. 4μl of test compound (100X solution in neat DMSO as 7 point curve ranging from $10^{-6}$ to $10^{-12}$M. final concentration) or DMSO (to define total binding) or a final concentration of 10μM fluoxetine in DMSO (to define non-specific binding, NSB) are added to wells; after this, 200μl of [N-Methyl-$^3$H]citalopram (Amersham Biosciences, 80 Ci/mmol) at the final concentration of 0.25nM in Assay Buffer, is added to all wells and finally the reaction is started by adding 200μl/well of membranes diluted 1:80 in Assay Buffer at concentration of about 2.5μg/well of protein. The reaction is carried out at room temperature for 2 hours and then stopped by rapid filtration through GF/B Unifilter 96-filterplate (Perkin-Elmer) pre-soaked in 0.5% polyethylenimmine (PEI) using a Perkin-Elmer FilterMat-196 harvester. Filterplate is washed 3 times with 1 ml/well ice-cold 0.9% NaCl solution. The plate is dried in an oven for 60 min at 50 °C then opaque bottom-seal is placed on the underside of the plate and 50μl of Microscint 20 (Perkin-Elmer) added to each well. Plate is sealed with a TopSeal and the radioactivity in the samples is counted for 4 min using TopCount liquid scintillation counter (Packard-Perkin-Elmer) and recorded as counts per minute (CPM). Competition binding assay for hNET may be conducted essentially as previously reported for hSERT in 96 well format and in a final assay volume of 400μl except for the use of hNET-LLCPK cell membranes (1:40 dilution i.e. 4.8μg of protein/well) and [$^3$H]nisoxetine as radioligand (1.5nM [N-methyl-$^3$H]nisoxetine, Amersham Biosciences, 84 Ci/mmol). 10μM desipramine is used for NSB.
Competition binding assay for hDAT may be conducted essentially as previously reported for hSERT and hNET in 96 well format and in a final assay volume of 400μl, except for the use of hDAT-LLCPK cell membranes (1:20 i.e. 9.6μgof protein/well) and [$^3$H]WIN-35,428 as radioligand (10nM [N-Methyl$^3$H]WIN-35,428, Perkin Elmer, 85.6 Ci/mmol). Furthermore, 10μM GBR-12909 is used for NSB and the incubation time of the binding reaction is 1 hour at room temperature.

**b) Scintillation Proximity Assay (SPA) for human DAT, NET and SERT binding**

• Transduction of HEK-293F cells with hSERT/hDAT/hNET BacMam viruses

The HEK-293F suspension cell line (Invitrogen) is routinely grown in 293-Freestyle Expression media (Invitrogen) in shake flask suspension culture. The culture is transduced with the appropriate transporter BacMam at a MOI (multiplicity of infection) of 100 virus particles per cell and incubated for 48hrs at 37°C, 5% $CO_2$in air, shaken at 90rpm in a humidified shaker incubator. The culture is then harvested by centrifugation at 1000g, 4°C, for 10 minutes and the cell pellet stored at -80°C until required.

• Preparation of BacMam hSERT/hDAT/hNET-HEL293F cell membranes

Transduced cell pellets are re-suspended to 10x volume with buffer-A (50mM HEPES, 1mM EDTA, 1mM leupeptin, 25ug/mL bacitracin, 1mM phenylmethylsulfonylfluoride, PMSF, 2μM pepstatin A, pH 7.7) and homogenised with 2x 15 second bursts in a glass Waring blender. The homogenate is then centrifuged for 20 minutes at 500g. Following this, the supernatant is pooled and centrifuged at 13,000g for 30 minutes. Pellets are then re-suspended to 4x original pellet volume with buffer-B (50mM TRIS pH 7.4, 130mM NaCl) and forced through a 0.8mm needle to give a homogeneous suspension. Membrane aliquots are stored at -80°C until required. The protein concentration is quantified by Bradford assay.

• SPA-binding assay protocol for hSERT, hNET, and hDAT

The affinity of the compounds of the invention to the hSERT, hNET or hDAT can be also assessed by using the [$^3$H]citalopram, [$^3$H]nisoxetine or [$^3$H]WIN-35,428 binding assays with the SPA technology on BacMam-recombinant human SERT, NET and DAT membranes produced as described before. With the SPA technology (GE Healthcare, Amersham) only transporter-bound radioactivity can elicit bead excitation thus no separation of the bound/ unbound radioligand is required.

The protocol for hSERT binding SPA is based on Trilux beta-counter (Wallac, Perkin-Elmer). Briefly, 0.5μl of test compound in neat DMSO (or 1μM fluoxetine as positive control) is added by 50μL of the SPA mixture, containing 2mg/mL SPA beads (Amersham RPNQ0001), 4μg/mL hSERT Bacmam membranes, 0.01% pluronic F-127, 2.5nM [$^3$H]citalopram in the assay buffer (20mM HEPES, 145mM NaCl, 5mM KCl, pH 7.3). Incubation are performed at room temperature for at least 2 hours. Counts are stable and could be read up to 3 days.
Alternatively, hDAT hNET and hSERT SPA-binding assays are performed by using a Viewlux beta-counter (Wallac, Perkin-Elmer) with imaging PS-WGA beads (Amersham RPNQ0260) in a final assay volume of 30μL and in a 384-well plate format (Greiner 781075). Briefly, 0.3μL of test compound in neat DMSO and 0% and 100% effect controls (DMSO for total binding and 10 or 1μM indatraline as positive control) are added to the wells by using a Hummingbird (Genomic Solutions), followed by the addition of 30μL of the SPA mixture, containing 1mg/mL SPA beads (hSERT) or 2mg/ml SPA beads (hDAT and hNET), 40μg/ml or 20μg/ml or 6 μg/ml of hDAT or hNET or hSERT BacMam membranes, 0.02% pluronic F-127, 10nM [$^3$H]WIN-35,428 or 10nM [$^3$H]nisoxetine or 3nM [$^3$H]citalopram for hDAT or hNET or hSERT binding SPA in the assay buffer (20mM HEPES, 145mM NaCl, 5mM KCl, pH 7.3-7.4). Incubation is performed at room temperature for at least 2 hours, best overnight in the dark. Bound radioactivity is recorded by using a 600s 6x binning and 613nm emission filter with the Viewlux instrument.

Compound affinity range for human transporters SERT, NET, and DAT

The compounds of formula (I)’ typically show pKi greater than 4.5 towards each of the three transporters SERT, NET and DAT. In one embodiment, the compounds of formula(I) typically show pKi greater than 5.5 for each of the three transporters. In another embodiment, the compounds of formula (I)’ typically show pKi greater than 6.5 for each of the three transporters. In a further embodiment, the compounds of formula (I)’ typically show pKi greater than 7.0 for each of the three transporters.
In one embodiment, the present invention provides compounds of formula (I)’ having a hSERT pKi comprised between 6.5 and 8.0.
In one embodiment, the present invention provides compounds of formula (I) having a hDAT pKi comprised between 6.0 and 7.5.
In one embodiment, the present invention provides compounds of formula (I)’ having a hNET pKi comprised between 6.0 and 7.5.
In one embodiment, the present invention provides compounds of formula (I)’ having a a hSERT pKi comprised between 6.5 and 8.0, a hNET pKi comprised between 6.0 and 7.5 and a hDAT pKi comprised between 6.0 and 7.5.
In another embodiment, the present invention provides compounds of formula (I)’ having a a hSERT pKi comprised between 7.0 and 8.0, a hNET pKi comprised between 6.5 and 7.0 and a hDAT pKi comprised between 7.0 and 7.5.

## Examples

The invention is further illustrated by the following examples.
In the procedures that follow, after each starting material, reference to a Preparation or Example by number is typically provided. This is provided merely for assistance to the skilled chemist. The starting material may not necessarily have been prepared from the batch referred to.
Where reference is made to the use of a "similar" or "analogous" procedure, as will be appreciated by those skilled in the art, such a procedure may involve minor variation, for example reaction temperature, reagent/solvent amount, reaction time, work-up conditions or chromatographic purification conditions.
Compounds are named using ACD/Name PRO 6.02 chemical naming software (Advanced Chemistry Development Inc., Toronto, Ontario, M5H2L3, Canada).
All temperatures refer to °C.
Proton Magnetic Resonance (NMR) spectra are typically recorded either on Varian instruments at 300, 400 or 500 MHz, or on a Bruker instrument at 300 and 400 MHz. Chemical shifts are reported in ppm (d) using the residual solvent line as internal standard. Splitting patterns are designed as s, singlet; d, doublet; t, triplet; q, quartet; m, multiplet; b, broad. The NMR spectra were recorded at a temperature ranging from 25 to 90°C. When more than one conformer was detected the chemical shifts for the most abundant one is reported.
Mass spectra (MS) are typically taken on a 4 II triple quadrupole Mass Spectrometer (Micromass UK) or on a Agilent MSD 1100 Mass Spectrometer, operating in ES (+) and ES (-) ionization mode or on an Agilent LC/MSD 1100 Mass Spectrometer, operating in ES (+) and ES (-) ionization mode coupled with HPLC instrument Agilent 1100 Series. In the mass spectra only one peak in the molecular ion cluster is reported.
Flash silica gel chromatography was carried out on silica gel 230-400 mesh (supplied by Merck AG Darmstadt, Germany) or over Varian Mega Be-Si pre-packed cartridges or over pre-packed Biotage silica cartridges.

In a number of preparations, purification was performed using either Biotage manual flash chromatography (Flash+) or automatic flash chromatography (Horizon or SP1) systems. All these instruments work with Biotage Silica cartridges.

The following abbreviations are used in the text: DCE= dichloroethane, Tlc refers to thin layer chromatography on silica plates, and dried refers to a solution dried over anhydrous sodium sulphate, r.t. (RT) refers to room temperature, Rt = retention time, DMSO = dimethyl sulfoxide; DCM - dichloromethane; DCE = dichloroethane; DME = dimethoxyethane; DMF = N,N'-dimethylformamide; MeOH = methanol; TEA = triethylamine; THF = tetrahydrofurane; AcOEt = ethyl acetate; $Et_2O$ = diethyl ether; SCX Cartridge = Strong Cation Exchange Cartridge; aminic cartridge: secondary amine functionalised silica cartridge; FC = flash chromatography..

**Preparation 1: 3-(3,4-dichlorophenyl)-2-propyn-1-ol (P1)**

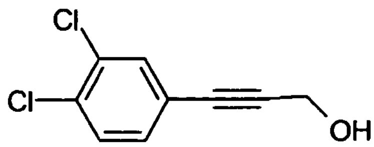

**Method A:** The title compound (2.94 g) was prepared in analogy to the method described in JOC 2005, 70, 4043-4053 starting from 3,4-dichloroiodobenzene (4 g, two preparation were carried out).

**Method B:** a mixture of 3,4-dichlorolodobenzene (300 mg), propargyl alcohol (128 μL), CuI (10 mg), $K_2CO_3$ (302 mg), $Pd(PPh_3)_4$ (12 mg) in DMF (2 mL) was irradiated with MicroWave at 100 °C for 20 min. Aqueous saturated solution $NH_4Cl$ was then added followed by DCM. After separation of the two phases the organic layer was dried and evaporated *in vacuo.* The crude product was purified by flash chromatography (eluting with cyclohexane / ethyl acetate 7/3) to give the title compound (40 mg).

**NMR ($^1H$, $CDCl_3$):** 7.58 (s, 1H), 7.41 (d, 1H), 7.27 (d, 1H), 4.52 (d, 2H), 1.75 (t, 1H)

**Preparation 2: 3-(3,4-dichlorophenyl)-2-propynal (P2)**

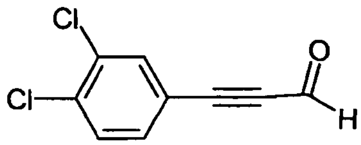

To a solution of 3-(3,4-dichlorophenyl)-2-propyn-1-ol (2.980 g, **P1**) in dry DCM (74 mL) and Dess-Martin periodinane (9.43 g) was added. The mixture was stirred at room temperature over night. $NaS_2O_3$ (19 g) and $NaHCO_3$ saturated solution were then added to the mixture and it was stirred at room temperature for 1 hour. Then the organic phase was separated and washed with brine. The organic layer was dried and concentrated under reduced pressure to give the crude title product (2.9 g) that was used without further purification.

**NMR ($^1H$, $CDCl_3$):** δ 9.48 (s, 1H), 7.73 (s, 1H), 7.55 (d, 1H), 7.42 (m, 1H).

**Preparation 3: (1*S*,5*S*/*1R*,*5R*)-1-(3,4-dichlorophenyl)bicyclo[3.1.0]hexan-3-one (P3)**

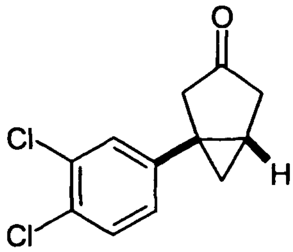

The title compound was prepared in analogy to the method described in J.Am.Chem.Soc. 2004, 126, 8654 from 3-(3,4-dichlorophenyl)-2-propynal (2.9 g) in 880 mg yield as an orange foam.
**NMR ($^1$H, $CDCl_3$)**: δ 7.45 (d, 1H), 7.28 (s, 1H), 7.11 (d, 1H), 2.89 (m, 2H), 2.70 (d, 1H), 2.42 (d, 1H), 2.05 (m, 1H), 1.38 (m, 1H), 0.72 (m, 1H).

**Preparation 4: (1*S*,5*S*/*1R*,*5R*)-1-(3,4-dichlorophenyl)bicyclo[3.1.0]hexan-3-one oxime (P4)**

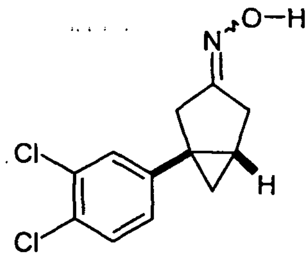

To a solution of hydroxylamine mono hydrate (1.26 g) and sodium acetate (2.3 g) in water (7 mL), a solution of (1S,5S/1R,5R)-1-(3,4-dichlorophenyl)bicyclo[3.1.0]hexan-3-one (0.860 g, **P3)** in ethanol (18 mL) was added at room temperature and the reaction mixture was stirred over night. After ethanol elimination under reduced pressure, the aqueous solution was extracted with DCM. The organic phase was dried and concentrated under reduced pressure to give the title compound (870 mg).
**NMR ($^1$H, $CDCl_3$)**: δ 7.40 (d, 1H), 7.26 (m, 1H), 7.05 (m, 1H), 3.33-2.60 (m, 4H), 1.89 (m, 1H), 1.15 (m, 1H), 0.68 (m, 1H). **MS(m/z):** 256 [MH]+.

**Preparation 5: (*1R,6R*/1*S*,6*S*)-1-(3,4-dichlorophenyl)-3-azabicyclo[4.1.0]heptan-4-one and (1R,6S/1S,6R)-6-(3,4-dichlorophenyl)-3-azabicyclo[4.1.0]heptan-4-one (P5)**

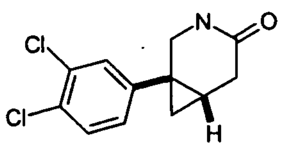
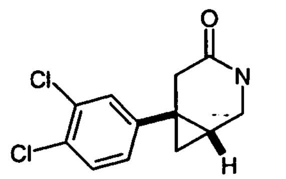

To a solution of (1S,5S/1R,SR)-1-(3,4-dichlorophenyl)bicyclo[3.1.0]hexan-3-one oxime (0.870 g, **P4**) in acetone (29 mL) sodium carbonate (solution 5% w/w in water, 25 mL) was added. Then, under vigorous stirring, a solution of tosyl chloride was added and the mixture stirred at room temperature for 30 minutes. The reaction mixture was heated at reflux for 2 h and at room temperature over night. After acetone elimination under reduced pressure, the residue was dissolved in $NaHCO_3$ saturated solution and it was extracted with DCM. The organic phase was dried and concentrated under reduced pressure. The crude was purified by flash chromatography (DCM/MeOH from 98/2 to 95/5) to give 640

mg of the mixture of title compounds.
**MS (m/z):** 256 [MH]+.

**Preparation 6: ethyl 5-(3,4-dichlorophenyl)-1-(phenylmethyl)-1,2,3,6-tetrahydro-4-pyridinecarboxylate (P6)**

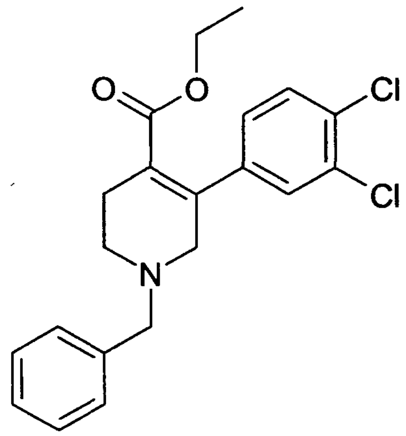

A solution of 4-butanoyl-3-oxo-1-(phenylmethyl)piperidinium chloride (3.0 g) in dry DMF (20 ml) was slowly added to a suspension of NaH (886 mg, 60% dispersed in mineral oil) in dry DMF (60 ml) at 0°C under nitrogen atmosphere. The mixture was stirred at 0°C for 1h and then *N*-phenyl-bis(trifluoromethanesulfonimide) (3.95 g) was added. The reaction mixture was stirred at room temperature for 4 h and then quenched with a saturated solution of $NH_4Cl$. The mixture was diluted with ethyl acetate, the organic extracted, washed with brine, dried and concentrated. Purification by chromatography eluting with a gradient 10-20% ethyl acetate/cyclohexane afforded 4-butanoyl-1-(phenylmethyl)-1,2,5,6-tetrahydro-3-pyridinyl trifluoromethanesulfonate (2.9 g). It was dissolved in DME (70 ml) and (3,4-dichlorophenyl)boronic acid (2.1 g), solid $K_2CO_3$ were added. The suspension was degassed with a steam on nitrogen and the $Pd(PPh_3)_3$ was added. The reaction mixture was heated to 80°C for 5 h. After cooling to room temperature, the solvent was evaporated under reduced pressure. The residue was partitioned between ethyl acetate (50 ml) and water (50 ml); the organic was washed with brine (20 ml), dried and concentrated *in vacuo.* Purification on silica gel (cyclohexane/ethyl acetate from 9/1 to 8/2) afforded 3:0 g of the title compound.
**NMR ($^1H$, $CDCl_3$):** δ ppm 7.21 - 7.43 (m, 7H), 6.95 - 6.99 (m, 1H), 3.97 (q, 2 H), 3.66 - 3.69 (m, 2 H), 3.19 - 3.23 (m, 2 H), 2.68 - 2.74 (m, 2 H), 2.55 - 2.62 (m, 2 H), 0.98 (t, 3 H)

**Preparation 7: ethyl 5-(3,4-dichlorophenyl)-1,2,3,6-tetrahydro-4-pyridinecarboxylate (P7)**

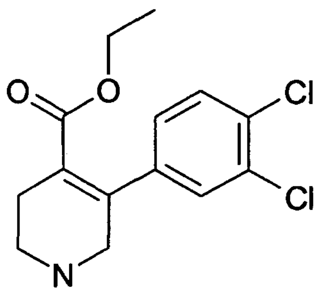

Ethyl 5-(3,4-dichlorophenyl)-1-(phenylmethyl)-1,2,3,6-tetrahydro-4-pyridinecarboxylate **(P6,** 2.0 g) was dissolved in dichloroethane (30 ml) and 1-chloroethyl chloridocarbonate (0.85 ml) was added. The reaction mixture was

heated to 50°C for 5 h and then slowly cooled to room temperature. 10 ml of methanol were added and the mixture was refluxed for 2 h. Evaporation of the volatiles afforded a residue that was partitioned between ethyl acetate and a saturated solution of $NaHCO_3$. The organic layer was washed with brine, dried and concentrated under vacuum. Purification by chromatography on silica gel (cyclohexane/ethyl acetate from 9/1 to 7/3) afforded 1.0 g of the title compound.
**NMR ($^1H$, $CDCl_3$):** δ ppm 7.41 (d, 1H), 7.26 (d, 1H), 7.00 (dd, 1H), 3.98 (q, 2H), 3.57 (t, 2 H), 3.08 (t, 2 H), 2.44 - 2.50 (m, 2 H), 0.99 (t, 3 H); **MS(m/z):** 300 [MH]+

**Preparation 8: 4-ethyl 1-(phenylmethyl) 5-(3,4-dichlorophenyl)-3,6-dihydro-1,4(2H)-pyridinedicarboxylate (P8)**

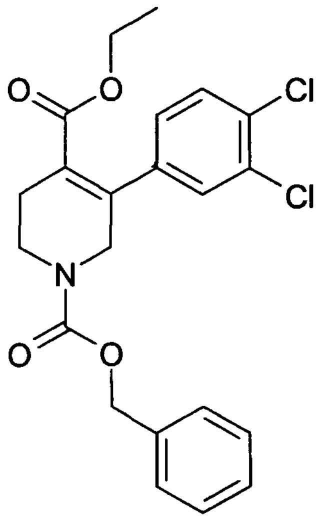

Ethyl 5-(3,4-dichlorophenyl)-1,2,3,6-tetrahydro-4-pyridinecarboxylate (**P7**, 1.0g) was dissolved in DCM and triethylamine (0.7 ml) was added. After cooling to 0°C, benzylchoroformate (0.52 ml) was added and the reaction mixture was stirred at this temperature for 1 h and then at room temperature for a further hour. The reaction mixture was quenched by addition of 0.1 N HCl; the organic phase was separated, washed with brine, dried and concentrated under vacuum affording 1.4g of the title compound.
**NMR ($^1H$, $CDCl_3$):** δ ppm 7.28 - 7.46 (m, 7H), 6.93 - 7.10 (m, 1H), 5.15 - 5.23 (m, 2H), 4.12 - 4.29 (m, 2 H), 4.00 (q, 2 H), 3.71 (t, 2H), 2.53 - 2.63 (m, 2 H), 0.99 (t, 3 H)

**Preparation 9: phenylmethyl 5-(3,4-dich)orophenyl)-4-(hydroxymethyl)-3,6-dihydro-1(2*H*-pyridinecarboxylate (P9)**

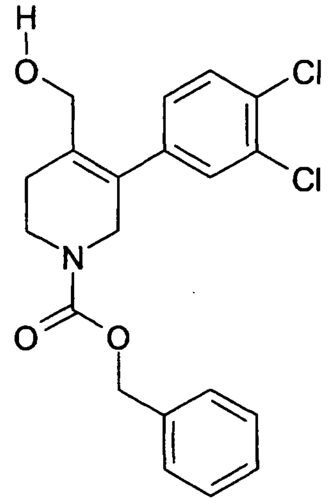

4-ethyl 1-(phenylmethyl) 5-(3,4-dichlorophenyl)-3,6-dihydro-1,4(2H)-pyridinedicarboxylate (**P8,** 1.4g) was dissolved in toluene (20 ml) and cooled to -20°C. $LiAlH_4$ 1.0M in THF (2.6 ml) was added dropwise at 20°C and the mixture was stirred at this temperature for 2h. The reaction mixture was quenched with a saturated solution of $NH_4Cl$ and the residue was diluted with ethyl acetate. The organic layer was dried and concentrated *in vacuo.* The residue was purified by chromatography on silica gel (cyclohexane/ethyl acetate from 3/1 to 1/1) to afford 0.79g of the title compound. **NMR ($^1H$, $CDCl_3$):** δ ppm 7.31 - 7.51 (m, 7H), 6.99 - 7.11 (m, 1H), 5.13 - 5.23 (m, 2H), 4.06 - 4.22 (m, 2 H), 3.92 - 4.05 (m, 2 H), 3.59 - 3.78 (m, J=5.87, 5.87 Hz, 2 H), 2.42 (s, 2 H)

**Preparation 10: 1,1-dimethylethyl 3-(3,4-dichlorophenyl)-3-hydroxy-1-piperidinecarboxylate (P10)**

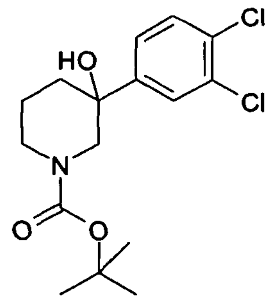

The Title compound was prepared in 4.38 g yield according to the method reported in Bioorganic & Medicinal Chemistry, 2001, 9 1349, starting from 1,1-dimethylethyl 3-oxo-1-piperidinecarboxylate (5 g) and bromo(3,4-dichlorophenyl)magnesium (55 mL, 0.5 M/THF).

**Preparation 11: 1,1-dimethylethyl 5-(3,4-dichlorophenyl)-3,6-dihydro-1(2*H*)-pyridinecarboxylate (P11)**

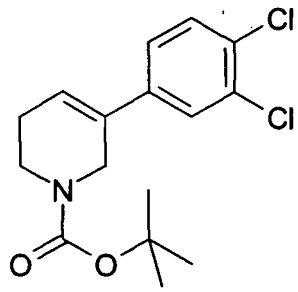

A mixture of 1,1-dimethylethyl 3-(3,4-dichlorophenyl)-3-hydroxy-l-piperidinecarboxylate (**P11,** 4.38 g) in trifluoroacetic acid (50 mL) was stirred at RT overnight, then was refluxed for 24 h. The reaction mixture was concentrated under reduced pressure, the residue was extracted with DCM, the organic phase was washed with saturated $NaHCO_3$, dried over $Na_2SO_4$ and the solvent evaporated under vacuum to give 3.61 g of the corresponding 3,6-dihydro intermediate. To a solution of this material (3.61 g) in DCM (100 mL), at RT, TEA (3 mL) and Boc anhydride (4,32 g) were subsequently added and the solution was stirred overnight. The reaction mixture was concentrated under reduced pressure and the crude product was purified by FC (eluting with cyclohexane/ethyl acetate from 1/0 to 8/2) to give 1.02 g of the Title compound.

**NMR ($^1$H, $CDCl_3$):** δ 7.32 - 7.55 (m, 2H) 7.14 - 7.24 (m, 1H) 6.13 - 6.32 (m, 1H) 4.10 - 4.33 (m, 2 H) 3.45 - 3.66 (m, 2 H) 2.22 - 2.44 (m, 2 H) 1.49 - 1.52 (m, 9 H)

**Example** 1: **(1S,6R/1R,6S)-1-(3,4-dichlorophenyl)-3-azabicyclo[4.1.0]heptane (E1)**

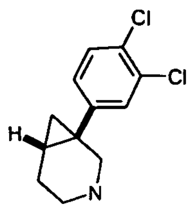

To a solution of (1R,6R/1S,6S)-1-(3,4-dichlorophenyl)-3-azabicyclo[4.1.0]heptan-4-one and (1R,6S/1S,6R)-6-(3,4-dichlorophenyl)-3-azabicyclo[4.1.0]heptan-4-one (640 mg, prepared as described for **P5**) in dry tetrahydrofurane (16 mL), borane (1 M in THF, 7.53 mL) was added under $N_2$ and the mixture heated at reflux for 3 h and at room temperature over night and then heated at reflux for 2h. The mixture was then cooled to 0 °C and methanol (8 mL) followed by hydrochloric acid (1 M/ether, 25mL) were cautiously added monitoring gas evolution and the solution stirred at room temperature over night. Solvents were then removed *in vacuo* and potassium carbonate (10% solution) was added to the residue. The aqueous layer was extracted with dichloromethane, then the organic phase was washed with a NaCl saturated solution, dried and concentrated under reduced pressure. The title compound was separated by aminic cartridge (eluting with cyclohexane/ethyl acetate from 9/1 to 7/3) to give the title compound in 207 mg yield.

**NMR ($^1$H, $CDCl_3$):** δ 7.38 (m, 2H), 7.15 (d, 1H), 3.31 (d, 1H), 3.11 (d, 1H), 2.85 (m, 1H), 2.55 (m, 1H), 2.05 (m, 1H), 1.70 (m, 1H), 1.34 (m, 1H), 1.02 (m, 1H), 0.92 (m, 1H); **MS(*m/z*):** 242 $[MH]^+$.

**Example 1** (207 mg) was separated into its enantiomers by semi-preparative HPLC using a chiral column Chiralpak AD-H, 25 x 4.6 cm, eluent A: n-hexane; B: ethanol 75/25, flow rate 0.8 mL/min, detection UV at 235 nm.

To a solution of E1 Enantiomer 1 (**E1e1**, **(1S,6R** or **1R,6S)-1-(3,4-dichlorophenyl)-3-azabicyclo[4.1.0]heptane,** Rt. = 6.83 min, ) in DCM was added 1 equivalent of HCl (1 M in $Et_2O$), the solvent evaporated *in vacuo* and the material thus obtained triturated with $Et_2O$ to give 89 mg of (1S,6R or 1R,6S)-1-(3,4-dichlorophenyl)-3-azabicyclo[4.1.0]heptane hydrochloride.

To a solution of E1 Enantiomer 2 (**E1e2, (1R,6S** or **1S,6R)-1-(3,4-dichlorophenyl)-3-azabicyclo[4.1.0]heptane, Rt.** = 9.97 min) in DCM was added 1 equivalent of HCl (1 M in $Et_2O$), the solvent evaporated *in vacuo* and the material thus

obtained triturated with $Et_2O$ to give 80 mg of (1R,6S or 1S,6R)-1-(3,4-dichlorophenyl)-3-azabicyclo[4.1.0]heptane hydrochloride.

**Example 2: (*1R*,6*R*)/(1S,6S)-1-(3,4-dichlorophenyl)-6-[(methyloxy)methyl]-3-azabicyclo[4.1.0]heptane (E2)**

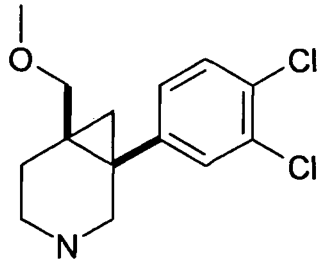

Phenylmethyl 5-(3,4-dichlorophenyl)-4-(hydroxymethyl)-3,6-dihydro-1 (2H)-pyridinecarboxylate (**P9,** 0.79g) in DCM (1 ml) was added at 0°C to a mixture of diethylzinc (1.0M in Hexane, 12 ml) and diiodomethane (1.95 ml) in DCM (40 ml) that was previously preformed and stirred at -20°C for 15 minutes. The reaction mixture was stirred at 0°C for 30 minutes then slowly warmed to room temperature and stirred at this temperature for 18 hours. The organic phase was then diluted with DCM (20 ml), quenched with 0.1 N HCl (20 ml) and the two phases vigorously stirred. The organic layer was then washed with brine (20 ml), dried and concentrated *in vacuo.* Purification by chromatography on silica gel (cyclohexane/ethyl acetate from 8/2 to 6/4) afforded impure phenylmethyl (1*R*,6*R*)/(1S,6S)-1-(3,4-dichlorophenyl)-6-(hydroxymethyl)-3-azabicyclo[4.1.0]heptane-3-carboxylate (120 mg) that was dissolved in DMF (15 ml) and added dropwise at 0°C to a suspension of NaH (60% in mineral oil, 18 mg) in DMF (5 ml). The mixture was stirred at 0°C for 30 min and then methyl iodide (35 $\mu$l) was added. The mixture was slowly warmed to room temperature. Additional NaH (60% in mineral oil, 27 mg) and methyl iodide (55 $\mu$l) were added and stirring was continued for 18 h. The reaction mixture was then partitioned between $Et_2O$ and $NH_4Cl$, the organic phase was washed with brine, dried and concentrated in vacuo. Purification by chromatography eluting with a gradient from 15 to 35% ethyl acetate/cyclohexane afforded impure phenylmethyl (1R,6R)/(1S,6S)-1-(3,4-dichlorophenyl)-6-[(methyloxy)methyl]-3-azabicyclo[4.1.0]heptane-3-carboxylate (90 mg) that was heated to 90°C in the presence of 6.0N HCl (3 ml) in dioxane (3 ml) for 18 h. The solution was made basic with NaOH 1.0M, the residue extracted with DCM and concentrated *in vacuo.* The crude product was purified first by SCX cartridge eluting with MeOH followed by 2.0M $NH_3$ in MeOH and then by chromatography eluting with 5% MeOH in DCM affording 20 mg of the title compound.

**NMR ($^1H$, $CDCl_3$):** $\delta$ ppm 7.46 - 7.49 (m, 1H), 7.37 (d, 1H), 7.22 (dd, 1H), 3.09 - 3.17 (m, 4 H), 2.97 - 3.06 (m, 4 H), 2.89 - 2.96 (m, 1H), 2.61 - 2.71 (m, 2 H), 2.00 - 2.09 (m, 1 H), 1.71 - 1.82 (m, 1H), 1.03 (d, 1H), 0.93 (d, 1H); **MS(m/z):** 286 [MH]+

**Example 3: (1R,6R)/(1S,6S)-1-(3,4-dichlorophenyl)-6-[(methyloxy)methyl]-3-azabicyclo[4.1.0]heptane hydrochloride (E3)**

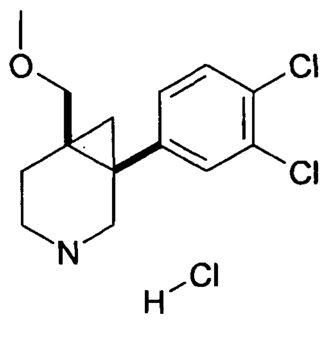

To a solution of **Example 2** (20 mg) in DCM was added 1 equivalent of HCl (1 M in $Et_2O$) the solvent evaporated

*in vacuo* and the material thus obtained triturated with $Et_2O$ to give 20 mg of the corresponding hydrochloride salt. **NMR ($^1$H, DMSO-d$_6$)** δ ppm 8.77 (br. s., 2 H), 7.77 (d, 1H), 7.59 (d, 1H), 7.42 (dd, 1H), 3.28 - 3.36 (m, 2 H), 3.10 - 3.18 (m, 1H), 3.01 (s, 3 H), 2.87 - 2.95 (m, 1H), 2.84 (d, 1H), 2.74 (d, 1H), 2.16 - 2.26 (m, 1H), 1.89 - 1.97 (m, 1H), 1.28 - 1.30 (m, 2 H).

19 mg of **E3** were separated into enantiomers by semi-preparative chiral SFC using a chiral column Chiralpak AS-H, 25 x 2.1 cm, modifier ethanol + 0.1% isopropylamine 7%, flow rate 22 mL/min, pressure 164 bar, temperature 36 °C, detection UV at 220 nm.

To a solution of Enantiomer 1 (**Rt.** = 10.83 min) in DCM was added 1 equivalent of HCl (1M in $Et_2O$), the solvent evaporated *in vacuo* and the material thus obtained triturated with $Et_2O$ to give 5.8 mg of the corresponding hydrochloride salt (**E4, (1R,6R or 1S,6S)-1-(3,4-dichlorophenyl)-6-[(methy)oxy)methy)]-3-azabicyclo[4.1.0]heptane hydrochloride**).

To a solution of Enantiomer 2 (**Rt.** = 12.09 min) in DCM was added 1 equivalent of HCl (1M in $Et_2O$), the solvent evaporated *in vacuo* and the material thus obtained triturated with $Et_2O$ to give 5.4 mg of the corresponding hydrochloride salt (**E5, (1S,6S** or **1R,6R)-1-(3,4-dichlorophenyl)-6-[(methyloxy)methyl]-3-azabicyclo[4.1.0]heptane hydrochloride).**

**Example 6: (1*R*,7*S*/1*S*,7*R*)-1-(3,4-dichlorophenyl)-7-[(methyloxy)methyl]-3-azabicyclo[4.1.0]heptane (E6)**

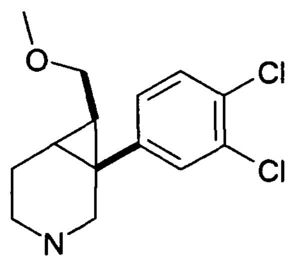

**Step A:** To a vigorous stirred solution of 1,1-dimethylethyl 5-(3,4-dichlorophenyl)-3,6-dihydro-1(2*H*)-pyridinecarboxylate (**P11**, 0.5 g) in DCM (7.5 mL), at RT, rhodium (II) acetate dimer (0.067 g) was added, followed by a solution of ethyldiazoacetate (0.24 mL) in DCM (1 mL) through a siring pump over 7 hours (the first half over 2h and the remaining in 5h). The resulting reaction mixture was then stirred overnight at RT, water was added, the organic phase was washed with brine, dried over Na2SO4 and the solvent removed in vacuo to give 0.23 g of a crude mixture mostly containing the starting material (**P12**).

**Step B:** To a vigorous stirred solution of 1,1-dimethylethyl 5-(3,4-dichlorophenyl)-3,6-dihydro-1(2*H*)-pyridinecarboxylate (**P11**) (0.48 g) together with the crude mixture from E6, Step A (0.23 g) in DCE (10 mL), at 50 °C, rhodium (II) acetate dimer (0.095 g) was added, followed by a solution of ethyldiazoacetate (0.34 mL) in DCE (1.5 mL) through a siring pump over 10 hours. The resulting reaction mixture was allowed to reach RT, water was added, the organic phase was washed with brine, dried over $Na_2SO_4$ and the solvent removed in vacuo. The crude product was purified by FC (eluting with cychlohexane/ethyl acetate from 1/0 to 9/1) to give 0.055 g of the corresponding cyclopropane intermediate. To a solution of this material (0.055 g) in toluene (1.4 mL), at -20 °C and under a nitrogen atmosphere, $LiAlH_4$ (0.532 mL, 1 M/THF) was added dropwise and the reaction was stirred for 1 h at -20 °C. Saturated $NH_4Cl$ was added and the reaction mixture was extracted with ethyl acetate. The organic phase was dried over $Na_2SO_4$ and the solvent evaporated under reduced pressure to give 40 mg of the corresponding crude alcohol intermediate. To a stirred solution of this material (40 mg) in DMF at 20 °C, NaH (5.6 mg, 60% in oil) was added in one portion and the reaction mixture was stirred for 0.5h, then MeI (0.013 mL) was added and the stirring continued for additional 4h. Saturated ammonium chloride and diethyl ether were added, the organic phase was washed with brine and evaporated to give a crude product that was dissolved in DCM (1mL). To this solution, at RT, trifluoroacetic acid was added and the reaction stirred for 2h, the solvent was evaporated under reduce pressure, the crude product was purified by semi-preparative liquid chromatography. The product obtained was dissolved in DCM (1mL) and passed through a SCX cartridge (eluting with methanol and 2N $NH_3$/methanol) obtaining 6 mg of the Title compound as a white foam.

**NMR ($^1$H, CDCl$_3$)**: δ 7.39 (d, 1H) 7.34 (d, 1H) 7.15 (dd, 1H) 4.01 (dd, 1 H) 3.90 (dd, 1 H) 3.43 (s, 3 H) 3.35 (d, 1 H) 3.10 (d, 1 H) 2.79 - 2.91 (m, 1 H) 2.53 - 2.64 (m, 1 H) 2.03 - 2.14 (m, 1 H) 1.62 - 1.83 (m, 1 H) 1.49 - 1.59 (m, 1 H) 1.33 - 1.42 (m, 1 H); **MS(*m/z*):** 286.03

## Claims

**1.** A compound of formula (I)' or a pharmaceutically acceptable salt or solvate thereof:

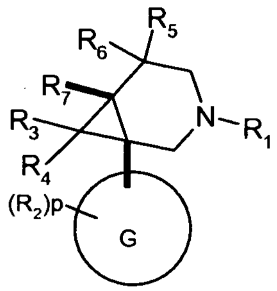

(I)'

wherein

G is selected from a group consisting of: phenyl, a 5- or 6-membered monocyclic heteroaryl group, or a 8- to 11-membered heteroaryl bicyclic group; such G may be substituted by $(R_2)_p$, which can be the same or different;
$R_1$ is hydrogen or $C_{1-4}$ alkyl;
$R_2$ is halogen, hydroxy, cyano, $C_{1-4}$alkyl, halo$C_{1-4}$alkyl, $C_{1-4}$alkoxy, halo$C_{1-4}$alkoxy, $C_{1-4}$alkanoyl and $SF_5$; or corresponds to $R_8$;
$R_5$ is hydrogen or $C_{1-4}$ alkyl;
$R_6$ is hydrogen or $C_{1-4}$ alkyl;
$R_7$ is selected in the group consisting of: hydrogen, fluorine, and $C_{1-4}$ alkyl; or corresponds to X, $X_1$, $X_2$ or $X_3$; wherein
X corresponds to:

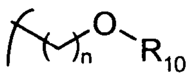

$X_1$ corresponds to:

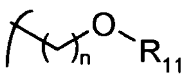

$X_2$ corresponds to:

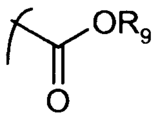

$X_3$ corresponds to:

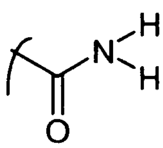

$R_3$ is hydrogen or $C_{1-4}$ alkyl; or corresponds to X or $X_1$;

$R_4$ is hydrogen or $C_{1-4}$ alkyl; or corresponds to X or $X_1$;
$R_8$ is a 5-6 membered heterocycle group, which may be substituted by one or two substituents selected from a group consisting of: halogen, cyano, $C_{1-4}$alkyl, halo$C_{1-4}$alkyl, $C_{1-4}$alkoxy and $C_{1-4}$alkanoyl;
$R_9$ is $C_{1-4}$alkyl;
$R_{10}$ is hydrogen , $C_{1-4}$alkyl, $C_{3-6}$cycloalkyl or $C_{3-6}$cycloalkyl$C_{1-3}$alkyl;
$R_{11}$ is halo$C_{1-2}$alkyl;
p is an integer from 0 to 5;
n is 1 or 2.

**2.** A compound as claimed in claim 1, which is a compound of formula (IC):

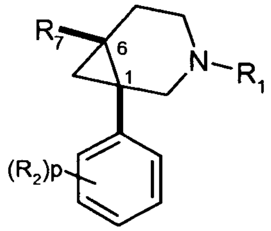

(IC)

wherein $R_1$, $R_2$, $R_7$ and p are as defined for compounds of formula (I)', or a pharmaceutically acceptable salt or solvate thereof.

**3.** A compound as claimed in claim 1 or 2, which is a compound of formula (ID):

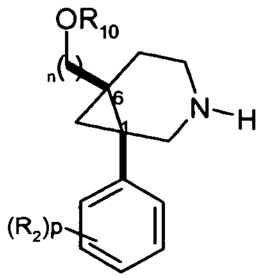

(ID)

wherein $R_{10}$, $R_2$, n and p are as defined for compounds of formula (I)', or a pharmaceutically acceptable salt or solvate thereof.

**4.** A compound as claimed in claim 1, which is a stereochemical isomer, having a single absolute configuration at stereogenic centers named 1 and 6, of formula (I)"

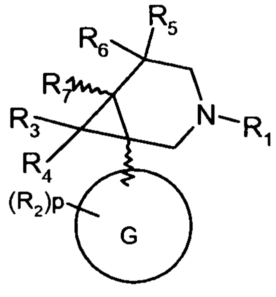

(I)"

wherein $R_1$, $R_2$, $R_3$, $R_4$, $R_5$, $R_6$, $R_7$ and p are defined as above for compounds of formula (I)', or a pharmaceutically

acceptable salt or solvate thereof.

5. A compound of formula (I)', as claimed in anyone of claims 1 to 4, selected from the list consisting of:

1S,6R/1R,6S)-1-(3,4-dichlorophenyl)-3-azabicyclo[4.1.0]heptane;
(1S,6R or 1R,6S)-1-(3,4-dichlorophenyl)-3-azabicyclo[4.1.0]heptane;
(1R,6S or 1S,6R)-1-(3,4-dichlorophenyl)-3-azabicyclo[4.1.0]heptane;
(1R,6R)/(1S,6S)-1-(3,4-dichlorophenyl)-6-[(methyloxy)methyl]-3-azabicyclo[4.1.0]heptane;
(1R,6*R* or 1S,6S)-1-(3,4-dichlorophenyl)-6-[(methyloxy)methyl]-3-azabicyclo[4.1.0]heptane;
(1S,6S or 1*R*,6*R*)-1-(3,4-dichlorophenyl)-6-[(methyloxy)methyl]-3-azabicyclo[4.1.0]heptane;
(1R,7*S*/1*S*,7*R*)-1-(3,4-dichlorophenyl)-7-((methyloxy)methyl]-3-azabicyclo[4.1.0]heptane; and pharmaceutically acceptable salts or solvates thereof.

6. Use of a compound as claimed in any of claims 1-5 in the manufacture of a medicament for the treatment of a condition for which inhibition of serotonin (5-HT), dopamine (DA) and norepinephrine (NE) is beneficial.

7. Use as claimed in claim 6, wherein the condition to be treated is depression.

8. A compound as claimed in any of claims 1-5 for use in therapy.

9. A compound as claimed in any of claims 1-5 for use in the treatment of a condition for which inhibition of serotonin (5-HT), dopamine (DA) and norepinephrine (NE) is beneficial.

10. A compound as claimed in any of claims 1-5 for use in the treatment of depression.

11. A pharmaceutical composition comprising a compound as claimed in any of claims 1-5 or a pharmaceutically acceptable salt or solvate thereof and a pharmaceutically acceptable carrier.

## Patentansprüche

1. Eine Verbindung der Formel (I)' oder ein pharmazeutisch verträgliches Salz oder Solvat davon:

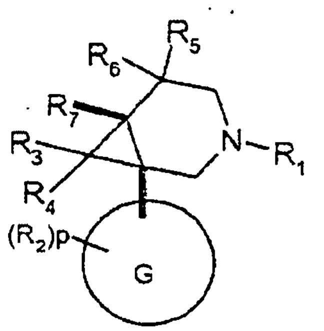

(I)'

wobei
G ausgewählt ist aus einer Gruppe bestehend aus Phenyl, einem 5- oder 6-gliedrigen monocyclischen Heteroarylrest oder einem 8- bis 11-gliedrigen bicyclischen Heteroarylrest; wobei ein solcher Rest G mit $(R_2)_p$, welches gleich oder verschieden sein kann, substituiert sein kann;
$R_1$ Wasserstoff oder $C_{1-4}$-Alkyl ist;
$R_2$ Halogen, Hydroxy, Cyano, $C_{1-4}$-Alkyl, Halogen-$C_{1-4}$-alkyl, $C_{1-4}$-Alkoxy, Halogen-$C_1$-$_4$-alkoxy, $C_{1-4}$-Alkanoyl und $SF_5$ ist oder $R_8$ entspricht;
$R_5$ Wasserstoff oder $C_{1-4}$-Alkyl ist;
$R_6$ Wasserstoff oder $C_{1-4}$-Alkyl ist;
$R_7$ ausgewählt ist aus der Gruppe bestehend aus Wasserstoff, Fluor und $C_{1-4}$Alkyl, oder $X_1$ $X_1$, $X_2$ oder $X_3$ entspricht;
wobei
x

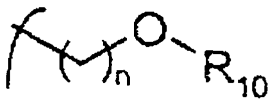

entspricht;

$X_1$

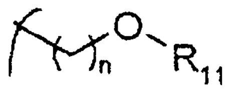

entspricht;

$X_2$

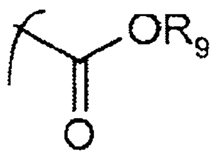

entspricht;

$X_3$

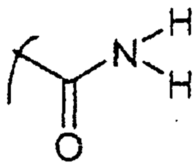

entspricht;

$R_3$ Wasserstoff oder $C_{1-4}$-Alkyl ist oder X oder $X_1$ entspricht;

$R_4$ Wasserstoff oder $C_{1-4}$-Alkyl ist oder X oder $X_1$ entspricht;

$R_8$ ein 5- bis 6-gliedriger Heterocyclusrest ist, welcher mit einem oder zwei Substituenten, ausgewählt aus einer Gruppe bestehend aus Halogen, Cyano, $C_{1-4}$-Alkyl, Halogen-$C_{1-4}$-alkyl, $C_{1-4}$-Alkoxy und $C_{1-4}$-Alkanoyl, substituiert sein kann;

$R_9$ $C_{14}$-Alkyl ist;

$R_{10}$ Wasserstoff, $C_{1-4}$-Alkyl, $C_{3-6}$-Cycloalkyl oder $C_{3-6}$-Cycloalkyl-$C_{1-3}$-alkyl ist;

$R_{11}$ Halogen-$C_{1-2}$-alkyl ist;

p eine ganze Zahl von 0 bis 5 ist;

n 1 oder 2 ist.

**2.** Eine Verbindung wie in Anspruch 1 beansprucht, welche eine Verbindung der Formel (IC) ist:

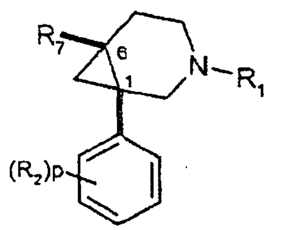

(IC)

wobei $R_1$, $R_2$, $R_7$ und p wie für die Verbindungen der Formel (I)' definiert sind, oder ein pharmazeutisch verträgliches Salz oder Solvat davon.

3. Eine Verbindung wie in Anspruch 1 oder 2 beansprucht, welche eine Verbindung der Formel (ID) ist:

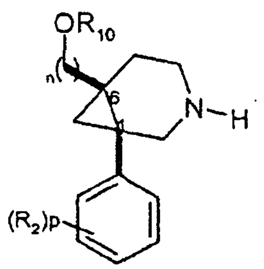

(ID)

wobei $R_{10}$, $R_2$, n und p wie für die Verbindungen der Formel (I)' definiert sind, oder ein pharmazeutisch verträgliches Salz oder Solvat davon.

4. Eine Verbindung wie in Anspruch 1 beansprucht, welche ein stereochemisches Isomer der Formel (I)" mit einer einzigen absoluten Konfiguration an den stereogenen Zentren, die als 1 und 6 bezeichnet sind, ist

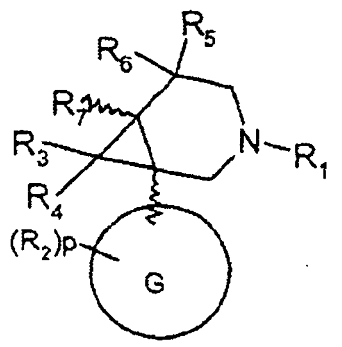

(I)"

wobei $R_1$, $R_2$, $R_3$, $R_4$, $R_5$, $R_6$, $R_7$ und p wie vorstehend für die Verbindungen der Formel (I)' definiert sind, oder ein pharmazeutisch verträgliches Salz oder Solvat davon.

5. Eine Verbindung der Formel (I)' wie in einem der Ansprüche 1 bis 4 beansprucht, ausgewählt aus der Liste bestehend aus:

(1S,6R/1R,6S)-1-(3,4-Dichlorphenyl)-3-azabicyclo[4.1.0]heptan;
(1S,6R oder 1R,6S)-1-(3,4-Dichlorphenyl)-3-azabicyclo[4.1.0]heptan;
(1R,6S oder 1S,6R)-1-(3,4-Dichlorphenyl)-3-azabicyclo[4.1.0]heptan;
(1R,6R)/(1S,6S)-1-(3,4-Dichlorphenyl)-6-[(methyloxy)methyl]-3-azabicyclo[4.1.0]-heptan;
(1R,6R oder 1S,6S)-1-(3,4-Dichlorphenyl)-6-[(methyloxy)methyl]-3-azabicyclo[4.1.0]-heptan;

(1S,6S oder 1R,6R)-1-(3,4-Dichlorphenyl)-6-[(methyloxy)methyl]-3-azabicyclo[4.1.0]-heptan;
(1R,7S/1S,7R)-1-(3,4-Dichlorphenyl)-7-[(methyloxy)methyl]-3-azabicyclo[4.1.0]heptan;
und pharmazeutisch verträgliche Salze oder Solvate davon.

**6.** Verwendung einer Verbindung wie in einem der Ansprüche 1 bis 5 beansprucht zur Herstellung eines Medikaments zur Behandlung eines Zustands, für den die Hemmung von Serotonin (5-HT), Dopamin (DA) und Norepinephrin (NE) vorteilhaft ist.

**7.** Verwendung wie in Anspruch 6 beansprucht, wobei der zu behandelnde Zustand Depression ist.

**8.** Eine Verbindung wie in einem der Ansprüche 1 bis 5 beansprucht zur Verwendung in der Therapie.

**9.** Eine Verbindung wie in einem der Ansprüche 1 bis 5 beansprucht zur Verwendung bei der Behandlung eines Zustands, für den die Hemmung von Serotonin (5-HT), Dopamin (DA) und Norepinephrin (NE) vorteilhaft ist.

**10.** Eine Verbindung wie in einem der Ansprüche 1 bis 5 beansprucht zur Verwendung bei der Behandlung von Depression.

**11.** Ein Arzneimittel, umfassend eine Verbindung wie in einem der Ansprüche 1 bis 5 beansprucht, oder ein pharmazeutisch verträgliches Salz oder Solvat davon und einen pharmazeutisch verträglichen Träger.

**Revendications**

**1.** Composé de formule (I)' ou sel ou solvat pharmaceutiquement acceptable de ce composé :

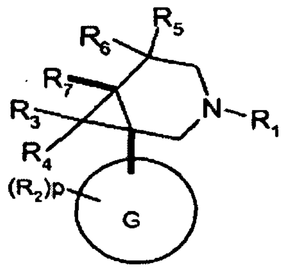

(I)'

où

G est choisi dans un groupe formé par : le groupe phényle, un groupe hétéroaryle monocyclique de 5 ou 6 chaînons, ou un groupe hétéroaryle bicyclique de 8 à 11 chaînons ; lequel G peut être substitué par $(R_2)_p$, qui peuvent être identiques ou différents ;
$R_1$ est l'hydrogène ou un groupe alkyle en $C_1$ à $C_4$;
$R_2$ est un halogène, un groupe hydroxyle, cyano, alkyle en $C_1$ à $C_9$, halogénoalkyle en $C_1$ à $C_4$, alkoxy en $C_1$ à $C_4$, halogénoalkoxy en $C_1$ à $C_4$, alcanoyle en $C_1$ à $C_4$ ou $SF_5$ ; ou correspond à $R_8$ ;
$R_5$ est l'hydrogène ou un groupe alkyle en $C_1$ à $C_4$ ;
$R_6$ est l'hydrogène ou un groupe alkyle en $C_1$ à $C_4$ ;
$R_7$ est choisi dans le groupe formé par : l'hydrogène, le fluor et un groupe alkyle en $C_1$ à $C_4$ ; ou correspond à X, $X_1$, $X_2$ ou $X_3$ ; où
X correspond à :

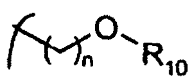

$X_1$ correspond à :

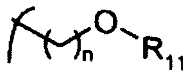

$X_2$ correspond à :

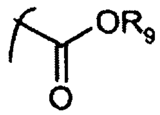

$X_3$ correspond à :

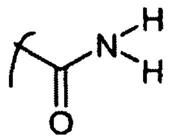

$R_3$ est l' hydrogène ou un groupe alkyle en $C_1$ à $C_4$ ; ou correspond à X ou $X_1$ ;
$R_4$ est l'hydrogène ou un groupe alkyle en $C_1$ à $C_4$ ; ou correspond à X ou $X_1$ ;
$R_8$ est un groupe hétérocyclique de 5 ou 6 chaînons, qui peut être substitué par un ou deux substituants choisis dans un groupe formé par : un halogène, un groupe cyano, alkyle en $C_1$ à $C_4$, halogénoalkyle en $C_1$ à $C_4$, alkoxy en $C_1$ à $C_4$ et alcanoyle en $C_1$ à $C_4$ ;
$R_9$ est un groupe alkyle en $C_1$ à $C_4$ ;
$R_{10}$ est l'hydrogène, un groupe alkyle en $C_1$ à $C_4$, cycloalkyle en $C_3$ à $C_6$ ou (cycloalkyle en $C_3$ à $C_6$) alkyle en $C_1$ à $C_3$ ; $R_{11}$ est un groupe halogénoalkyle en $C_1$ ou $C_2$ ;
p est un nombre entier de 0 à 5 ;
n est 1 ou 2.

2. Composé selon la revendication 1, qui est un composé de formule (IC) :

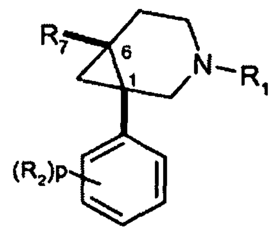

(IC)

où $R_1$, $R_2$, $R_7$ et p sont tels que définis pour les composés de formule (I)', ou sel ou solvat pharmaceutiquement acceptable de ce composé.

3. Composé selon la revendication 1 ou 2, qui est un composé de formule (ID) :

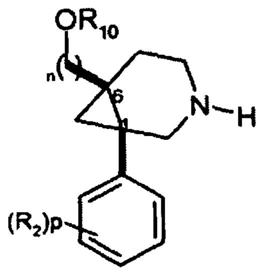

(ID)

où $R_{10}$, $R_2$, n et p sont tels que définis pour les composés de formule (I)', ou sel ou solvat pharmaceutiquement acceptable de ce composé.

4. Composé selon la revendication 1, qui est un isomère stéréochimique, ayant une configuration absolue unique au niveau des centres stéréogènes nommés 1 et 6, de formule (I) "

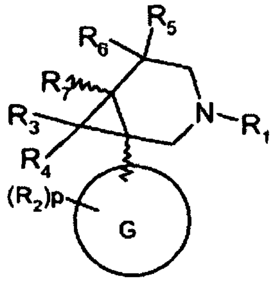

(I)"

où $R_1$, $R_2$, $R_3$, $R_4$, $R_5$, $R_6$, $R_7$ et p sont tels que définis ci-dessus pour les composés de formule (I)', ou sel ou solvat pharmaceutiquement acceptable de ce composé.

5. Composé de formule (I)' selon l'une quelconque des revendications 1 à 4, choisi dans la liste formée par les composés suivants :

(1*S*,6*R*/1*R*,6*S*)-1-(3,4-dichlorophényl)-3-azabicyclo[4.1.0]-heptane ;
(1*S*,6*R* ou 1*R*,6*S*)-1-(3,4-dichlorophényl)-3-azabicyclo[4.1.0]-heptane ;
(1*R*,6*S* ou 1*S*,6*R*)-1-(3,4-dichlorophényl)-3-azabicyclo[4.1.0]-heptane ;
(1*R*,6*R*)/(1*S*,6*S*)-1-(3,4-dichlorophényl)-6-[(méthyloxy)-méthyl]-3-azabicyclo[4.1.0]heptane ;
(1*R*,6*R* ou 1*S*,6*S*)-1-(3,4-dichlorophényl)-6-[(méthyloxy)-méthyl]-3-azabicyclo[4.1.0]heptane ;
(1*S*,6*S* ou 1*R*,6*R*)-1-(3,4-dichlorophényl)-6-[(méthyloxy)-méthyl]-3-azabicyclo[4.1.0]heptane ;
(1*R*,7*S*/1*S*,7*R*)-1-(3,4-dichlorophényl)-7-[(méthyloxy)méthyl]-3-azabicyclo[4.1.0]heptane ;
et leurs sels ou solvats pharmaceutiquement acceptables.

6. Utilisation d'un composé tel que revendiqué dans l'une quelconque des revendications 1 à 5 dans la fabrication d'un médicament destiné au traitement d'un état pathologique dans lequel l'inhibition de la sérotonine (5-HT), de la dopamine (DA) et de la norépinéphrine (NE) est salutaire.

7. Utilisation selon la revendication 6, dans laquelle l'état pathologique à traiter est la dépression.

8. Composé selon l'une quelconque des revendications 1 à 5, pour son utilisation en thérapie.

9. Composé selon l'une quelconque des revendications 1 à 5, pour son utilisation dans le traitement d'un état pathologique dans lequel l'inhibition de la sérotonine (5-HT), de la dopamine (DA) et de la norépinéphrine (NE) est salutaire.

10. Composé selon l'une quelconque des revendications 1 à 5, pour son utilisation dans le traitement de la dépression.

11. Composition pharmaceutique comprenant un composé tel que revendiqué dans l'une quelconque des revendications 1 à 5 ou un sel ou solvat pharmaceutiquement acceptable de ce composé, et un support pharmaceutiquement acceptable.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **T.W. Greene ; P.G.M. Wuts.** Protective groups in organic synthesis. John Wiley & sons, 1991
- **P.J. Kocienski.** Protecting Groups. Georg Thieme Verlag, 1994
- *J. Am. Chem. Soc.,* 2004, vol. 126, 8654
- *JOC,* 2005, vol. 70, 4043
- Diagnostic and Statistical Manual of Mental Disorders. American Psychiatric Association
- **H. Gu ; S.C. Wall ; G. Rudnick.** *J. Biol. Chem.,* 1994, vol. 269, 7124-7130
- **Condreay JP et al.** *Proc. Natl. Acad. Sci. USA,* 1999, vol. 96, 127-132
- **Hassan NJ et al.** *Protein Expression and Purification,* 2006, vol. 47 (2), 591-598
- **Cheng ; Prusoff.** *Biochem. Pharmacol.,* 1973, vol. 22, 3099
- **Michael J. Owens et al.** Neurotrasmitter receptor and transporter binding profile of antidepressants and their metabolites. *JPET,* 1997, vol. 283, 1305-1322
- **Per Allard ; Jan O. Marcusson ; Svate B. Ross.** [3H]WIN-35,428 binding in the human brain. *Brain Res.,* 1996, vol. 706, 347-350
- *JOC,* 2005, vol. 70, 4043-4053
- *J.Am.Chem.Soc,* 2004, vol. 126, 8654
- *Bioorganic & Medicinal Chemistry,* 2001, vol. 9, 1349